# EUROPÄISCHE PATENTANMELDUNG

(11) **EP 0 547 631 A2**
(43) Veröffentlichungstag der Anmeldung: **23.06.1993**
(21) Anmeldenummer: 92121630.5
(22) Anmeldetag: 18.12.1992
(51) Int. Cl.: C12N 15/13, C12P 21/08

(54) **Immunoglobulin-Expressionsvektor und damit hergestellte Antikörper**

(30) Priorität: 19.12.1991 DE 4142077
(71) Anmelder: BOEHRINGER MANNHEIM GMBH, D-68298 Mannheim (DE)
(72) Erfinder: Kaluza, Brigitte, Dr., W-8173 Bad Heilbrunn (DE); Betzl, Gisela, W-8122 Penzberg (DE); Weidle, Ulrich Heinz, Dr., W-8000 München 2 (DE)
(74) Vertreter: Huber, Bernhard, Dipl.-Chem.

(57) **Zusammenfassung**

Die vorliegende Erfindung betrifft ein Verfahren zur Herstellung eines Immunglobulin-Expressionsvektors, der die Expression von Immunglobulinen ohne Sequenzveränderungen in den kodierenden Regionen erlaubt. Ferner betrifft die Erfindung neue Basisvektoren zur Herstellung von Expressionsvektoren für Immunglobuline.

## Beschreibung

Die vorliegende Erfindung betrifft ein Verfahren zur Herstellung eines Immunglobulin-Expressionsvektors, der die Expression von Immunglobulinen ohne Sequenzveränderungen in den kodierenden Regionen erlaubt.

Die Herstellung rekombinanter Antikörper hat zunehmend praktische Bedeutung gewonnen. Dennoch waren die bislang hierfür zur Verfügung stehenden Technologien nicht in allen Punkten befriedigend. Ein erster essentieller Schritt bei der Konstruktion rekombinanter Antikörper ist die Klonierung von variablen Regionen der gewünschten Spezifität. Zwar wurde dieser Schritt mit Einführung der "polymerase chain reaction" (PCR) entscheidend vereinfacht, doch blieb das Problem bestehen, daß eine DNA-Region mit a priori unbekannter Sequenz durch zwei möglichst sequenzspezifische Primer amplifiziert werden sollte. Ein weiteres Problemfeld stellt die Expression rekombinanter Antikörper dar. Komplette Antikörper-Moleküle mit einer für Anwendung am Menschen kompatiblen Glycosylierung werden bevorzugt in lymphoiden Säugerzellen hergestellt ("Transfectoma-Linien"). Um hier akzeptable Expressionsleistungen zu erzielen, müssen komplexe Expressionsvektoren mit der Struktur von Immunglobulin-Genen verwendet werden. Bislang war es mit praktisch vertretbarem Aufwand nicht möglich, in solche Vektoren Antikörper-V-Regionen zu inserieren, ohne (wenn auch geringfügige) Veränderungen in deren Sequenz zu erzeugen.

Das in der vorliegenden Erfindung beschriebene Verfahren gestattet es erstmals, die variablen Regionen von Immunglobulin-Genen unter Verwendung von zwei völlig sequenzspezifischen, aber universell anwendbaren Primern zu amplifizieren. Weiterhin beinhaltet dieses Verfahren Expressionsvektoren, in die die Klonierung der so amplifizierten V-Regionen möglich ist, ohne daß Sequenzveränderungen in ihren kodierenden Regionen erfolgen. Gleichzeitig ist mit Hilfe dieser Expressionsvektoren die Produktion rekombinanter Antikörper mit den so klonierten V-Regionen in lymphoiden Säugerzellen mit besonders hoher Ausbeute möglich.

Zur Klonierung von Immunglobulin-V-Regionen stehen zum einen die klassischen Verfahren der Genklonierung zur Verfügung, die jedoch langwierig und aufwendig sind (F.Sambrook, E.F. Fritsch und T. Maniatis: Molecular Cloning - a Laboratory Manual, second edition, Cold Spring Harbor, New York 1989).

Eine weitaus schnellere Klonierung ist durch Anwendung der Polymerase-Kettenreaktion (polymerase chain reaction, PCR) möglich. Bei diesem Verfahren wird mit Hilfe von zwei meist synthetischen Oligonukleotiden, die zu flankierenden Sequenzen der zu amplifizierenden DNA komplementär sind, in einem sich wiederholenden Replikationsvorgang die dazwischenliegende DNA amplifiziert und anschließend kloniert. Eine Voraussetzung für die Anwendung der PCR ist jedoch die Kenntnis von Sequenzen, die beiden Seiten der zu klonierenden DNA-Region benachbart sind, damit die benötigten homologen Oligonukleotide (Primer) synthetisiert werden können.

Es wurde bereits versucht, die Methodik der PCR auf die Klonierung von Immunglobulin-V-Regionen anzuwenden. Einige Verfahren verwenden hierzu die mRNA, andere die genomische DNA derjenigen Zellen, die das gesuchte Immunglobulin-Gen exprimieren.

Die bislang beschriebenen Verfahren zur Klonierung von Immunglobulin-V-Regionen ausgehend von mRNA verwenden in allen Fällen zumindest einen Primer, der keine vollständige Homologie zu dem gesuchten Genabschnitt aufweist (Larrick, J.W., Danielsson, L., Brenner, C.A., Wallace, E.F., Abrahamson, M., Fry, K.E., Borrebaeck, C.A.K. (1989), Biochem. Biophys. Res. Com. 160, 1250 - 1256. Orlandi, R., Güssow, D.H., Jones, P.T., Winter, G. (1989), Proc.Natl. Acad. Sci. USA 86, 3833 - 3837. Sastry, L., Alting-Mees, M., Huse, W.D., Short, J.M., Sorge, J. A., Hay, B.N., Janda, K.D., Benkovic, S.J., Lerner, R.A. (1989), Proc. Natl. Acad. Sci. USA 86, 5728 - 5732). Ausgehend von der Beobachtung, daß bei Antikörpern die Sequenzen am 5'-Ende der V-Region oder im Bereich des Signalpeptides eine gewisse Konservierung aufweisen, wurden hier Primer verwendet, die mit einer entsprechenden Wahrscheinlichkeit zu diesen Bereichen homolog sind. Derartige Verfahren sind jedoch zum einen nicht universell anwendbar, da auf diese Weise atypische V-Regionen nicht effizient amplifiziert werden können. Außerdem verändert die Verwendung von nicht vollständig homologen Primern innerhalb der V-Region die Sequenz des Amplifizierungsprodukts im Bereich der Primerbindungsstelle, so daß die ursprüngliche Sequenzinformation im klonierten Fragment nicht mehr enthalten ist. Es wurde versucht, diese Nachteile zu kompensieren, indem eine große Zahl von Primern eingesetzt wurde, die möglichst spezifisch für die einzelnen Familien der Immunglobulin-V-Regionen sind; der mit diesem Verfahren verbundene extreme Aufwand ist aber offensichtlich (Marks J.D., Tristem M., Karpas, A. und Winter, G. (1991), Eur. J. Immunol. 21, 985 - 991).

Ein weiteres Verfahren geht von genomischer DNA aus (Zwickl, M., Zaninetta, D., McMaster, G.K., Hardman, N. (1990), J. Immunol. Meth. 130, 49 - 55). Hier wird zunächst durch Southern-Analyse eine Restriktionskartierung des zu klonierenden Immunglobulin- Gens durchgeführt. Anschließend wird ein geeignetes Restriktionsfragment durch Ligation zyklisiert und durch inverse PCR (Triglia, T., Peterson, M.G., Kemp, D.J. (1988), Nucl. Acids Res. 16, 8186) das V-Region haltige Segment amplifiziert. Die Nachteile dieses Verfahrens sind sein hoher Aufwand (infolge der Southern-Analyse) und die Tatsache, daß hier notgedrungen relativ große DNA-Fragmente amplifiziert werden müssen, was technisch anspruchsvoll und kaum routinemäßig anwendbar ist. In einem alternativen Verfahren (EP-A 0 481 502) wird vorgeschlagen, als erstes die Sequenz der mRNA des zu klonierenden Immunglobulins direkt zu bestimmen. Mit deren Kenntnis werden dann Primer entworfen, die eine Klonierung des gesuchten Fragmentes ausgehend von genomischer DNA gestatten. Auch dieses Verfahren erfordert einen hohen experimentellen Aufwand, wodurch es für Routineanwendungen kaum in Frage kommt.

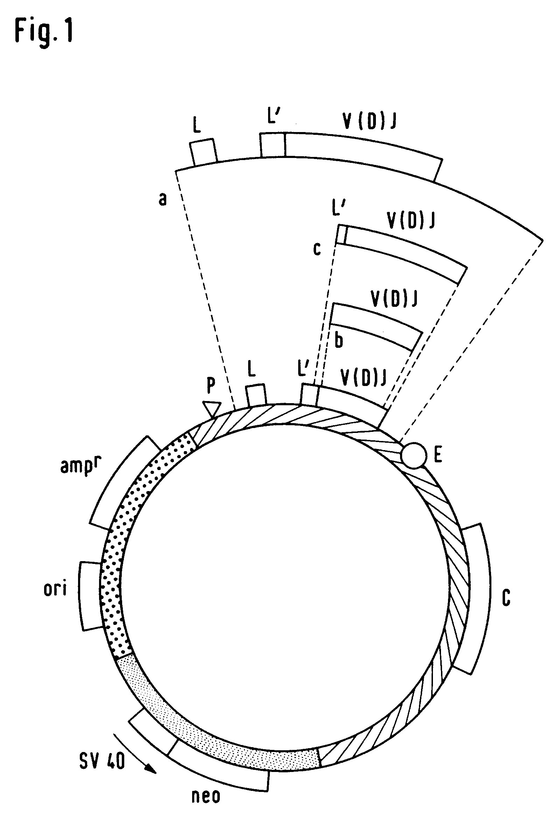

Zur Expression der klonierten Immunglobulin-V-Regionen in lymphoiden Säugerzellen haben sich bislang vor allem solche Vektoren hinsichtlich der Expressionsleistung bewährt, die in ihrem Aufbau die Struktur von Immunglobulin-Genen besitzen. In diese Vektoren können jedoch nur solche V-Regionen ohne Sequenzveränderung direkt eingesetzt werden, die als genomische Fragmente kloniert wurden (Abb. 1, Version "a"). Derartige genomische Fragmente sind aber, wie oben dargelegt, nur unter größeren Schwierigkeiten erhältlich. Von mRNA oder cDNA amplifizierte Fragmente enthalten bei den bisher angewandten Verfahren (siehe oben) in der Regel Schnittstellen für Restriktionsendonukleasen, die innerhalb des für die V-Region kodierenden Bereichs liegen und eine rastergerechte Insertion in das V(D)J-Exon eines Basisvektors (Expressionskassette) gestatten (Abb. 1, Version "b"). Dies Verfahren ist einfach und schnell, führt aber zu Veränderungen in der Aminosäuresequenz der exprimierten V-Region.

Aufgabe der vorliegenden Erfindung war es somit, ein Verfahren zur Klonierung der V-Region eines Immunglobulins bereitzustellen, bei dem die Nachteile des Standes der Technik mindestens teilweise beseitigt sind und mit dem eine Expression des klonierten Immunglobulins ohne Sequenzveränderungen in den kodierenden Regionen möglich ist.

Die erfindungsgemäße Aufgabe wird gelöst durch ein Verfahren zur Herstellung eines Immunglobulin-Expressionsvektors durch Klonierung eines DNA-Fragments, das einen für die komplette exprimierte V-Region eines ausgewählten Immunglobulins kodierenden Sequenzbereich enthält, in einen Basisvektor, der einen Promotor, die konstanten Immunglobulingen-Sequenzen und geeignete Restriktionsschnittstellen zur Insertion des oben genannten DNA-Fragments enthält, wobei die Klonierung derart erfolgt, daß ein Expressionsvektor mit einem operativen Immunglobulingen gebildet wird, wobei man zur Klonierung ein DNA-Fragment verwendet, das vor dem 5'-Ende des für die komplette exprimierte V-Region kodierenden Sequenzbereichs eine Region enthält, die für den vollständigen C-terminalen Abschnitt eines Immunglobulin-Leaderpeptids oder einen 3'-seitigen Teil davon kodiert, und daß das Fragment direkt hinter dem 3'-Ende des für die komplette exprimierte V-Region kodierenden Sequenzbereichs endet.

Die Region, "die für den vollständigen C-terminalen Abschnitt eines Immunglobulin-Leaderpeptids kodiert", ist derjenige Abschnitt aus dem Exon 2 eines Immunglobulingens, der nicht für die exprimierte V-Region kodiert. Dies bedeutet, daß das zur Klonierung verwendete DNA-Fragment genau dem vollständigen Immunglobulin - Exon 2, gegebenenfalls ohne einen 5'-seitigen Teil des Leaderabschnitts entspricht. Die für den C-terminalen Leaderabschnitt (im folgenden auch als L'-Segment bezeichnet) kodierende Nukleotidsequenz ist vorzugsweise 10 bis 15 Bp lang. Diese Region entspricht im allgemeinen nicht genau der authentischen Leadersequenz aus dem ausgewählten Immunglobulingen, sondern sie weist eine Restriktionsstelle zur Klonierung in den Basisvektor auf.

Bei dem erfindungsgemäßen Verfahren verwendet man einen Basisvektor, der für konstante Regionen der schweren oder/und der leichten Kette kodierende Immunglobulingen-Sequenzen, vorzugsweise einschließlich der jeweiligen nicht-kodierenden Intronsequenzen, enthält. Die konstanten Regionen sind vorzugsweise humanen Ursprungs. Beispiele für konstante Regionen einer schweren Kette sind γ-, µ-, δ-, ε- oder α-Immunglobulin-Sequenzen. Beispiele für konstante Regionen einer leichten Kette sind k- oder λ-Immunglobulin-Sequenzen. Derartige Sequenzen sind aus öffentlich zugänglichen Datenbanken erhältlich.

Unter dem Begriff der "kompletten exprimierten V-Region" ist derjenige Bereich eines Immunglobulingens zu verstehen, der bei einer leichten Kette der VJ-Region und bei einer schweren Kette der VDJ-Region entspricht. In einer bevorzugten Ausführungsform der vorliegenden Erfindung erfolgt die Gewinnung des für die V-Region des Immunglobulingens kodierenden DNA-Fragments durch die folgenden Schritte:
(a) Isolierung von RNA aus einer Zelle, die das ausgewählte Immunglobulingen exprimiert, und Umschreiben der RNA in cDNA mit Hilfe einer Reversen Transkriptase,
(b) Zyklisierung der cDNA durch Ligation in verdünnter Lösung,
(c) Amplifizierung der zyklisierten cDNA oder eines Abschnitts davon mit Hilfe von 2 Primern (1) und (2), die aus der konstanten Region des ausgewählten Immunglobulingens stammen, wobei die Primer (1) und (2) so gewählt werden, daß das resultierende Amplifizierungsprodukt mindestens einen Abschnitt aus dem Exon 2 des Immunglobulingens, der für das C-terminale Ende eines Immunglobulin-Leaderpeptids kodiert, sowie die komplette exprimierte V-Region des ausgewählten Immunglobulingens enthält,
(d) vollständige oder teilweise Sequenzierung des Amplifizierungsprodukts aus (c),
(e) Konstruktion von 2 Primern (3) und (4) anhand der aus (d) gewonnenen Sequenzinformation, wobei der Primer (3) innerhalb der für den C-terminalen Abschnitt des Immunglobulin-Leaderpeptids kodierenden Region und der Primer (4) im Bereich des 3'-Endes der exprimierten V-Region an das Amplifizierungsprodukt aus (c) bindet,
(f) Amplifizierung des Amplifizierungsprodukts aus (c) oder einer entsprechenden RNA bzw. cDNA aus (a) mit Hilfe der Primer (3) und (4) und
(g) gegebenenfalls Restriktionsschnitt des Amplifizierungsprodukts aus (f) zur Erzeugung passender Enden für die Klonierung in den Basisvektor.

Der oben genannten Ausführungsform der vorliegenden Erfindung liegt die Kombination dreier Erkenntnisse zugrunde:
1) Durch ein Amplifizierungsverfahren, vorzugsweise eine inverse PCR, und durch die Auswahl geeigneter Primer (1) und (2) aus der (bekannten) C-Region des Immunglobulingens wird die Klonierung und Sequenzermittlung einer beliebigen Immunglobulin-V-Region in voller Länge und in völlig unveränderter Form ermöglicht.
2) Bei genauer Analyse der Leaderpeptid-Sequenzen aus dem von Exon 2 des Immunglobulingens kodierten C-terminalen Leaderpeptidabschnitt (L'-Segment) zeigte es sich, daß durch entsprechende Mutagenese Schnittstellen für Restriktions-Endonukleasen in diese Sequenzen eingebracht werden können, die weder das Entfernen des Introns 1 aus dem primären Transkript des Immunglobulingens durch Splicing stören, noch die Aminosäuresequenz des Leaderpeptids in einer Weise verändern, die seine biologische Funktion, d.h. insbesondere Sekretion des Immunglobulins aus der Zelle und Abspaltung des Leaderpeptids von der reifen Antikörper-Kette, unterbindet. Dabei ist unter dem L'-Segment bzw. dem C-terminalen Abschnitt derjenige Teil des Leaderpeptids zu verstehen, der vom Exon der V-Region des Immunglobulingens (Exon 2) kodiert wird. Mit Hilfe der durch die vollständige oder teilweise Sequenzierung des unter Verwendung der Primer (1) und (2) erhaltenen Amplifizierungsprodukts gewonnenen Information über die V-Region kann nun ein Primer (Primer (3)) entworfen werden, der die Amplifikation einer kompletten V-Region mit eben dieser Schnittstelle am 5'-Ende gestattet, so daß sie hierdurch in eine Immunglobulin-Genkassette bzw. in einen Basisvektor einzufügen ist.
3) Eine genaue Analyse der DNA-Sequenzen am Übergang von Exon 2 (kodierend für das L'-Segment des Leaderpeptids und die V-Region) und Intron 2 führte zu der Erkenntnis, daß zum einen durch stumpf schneidende Restriktions-Endonukleasen ein Immunglobulin-Expressionsvektor konstruierbar ist, der präzise an der Intron-Exon-Grenze geöffnet werden kann. Zum anderen kann mit Hilfe der Sequenzinformation über das Amplifikationsprodukt der Primer (1) und (2) ein Primer (4) entworfen werden, der mit dem 3'-Bereich der exprimierten V-Region hybridisiert und, z.B. durch Anwendung von PCR, die Amplifizierung eines DNA-Fragments erlaubt, welches präzise mit der letzten Base des V-Region-Exons endigt.

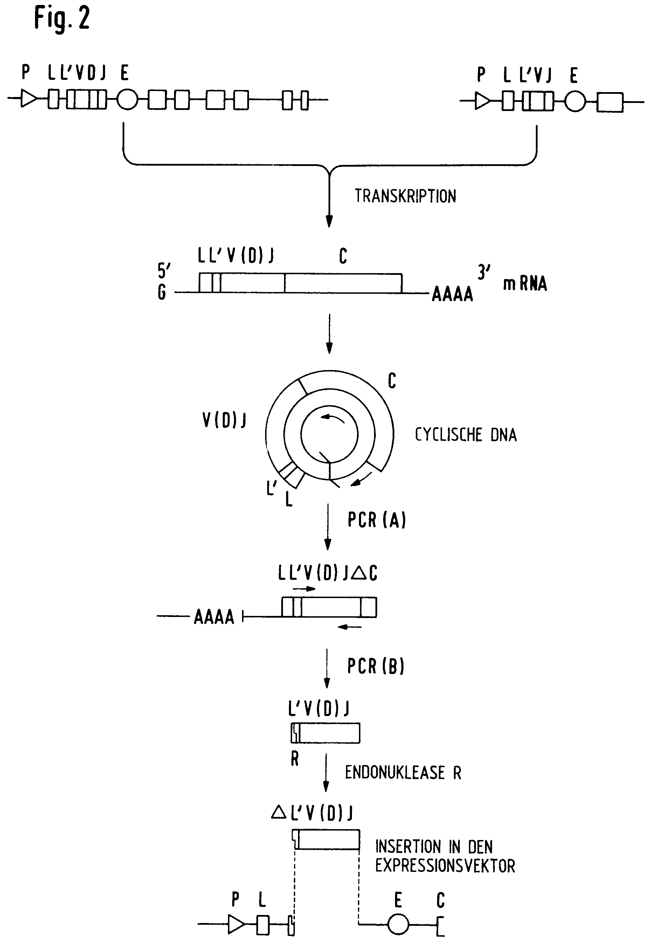

Die Kombination dieser drei Erkenntnisse ermöglicht ein rasches universelles Verfahren zur Klonierung und Expression von Immunglobulin-V-Regionen ohne jegliche Veränderung in ihren für den reifen Antikörper kodierenden Bereichen. In Abb. 2 sind die einzelnen Schritte zur Gewinnung von DNA-Fragmenten, die zur Klonierung in einem Basisvektor verwendet werden, schematisch dargestellt.

Der erste Schritt dieses Prozesses beinhaltet zunächst, ausgehend von mRNA, eine direkte Klonierung der V-Regionen von schwerer und leichter Kette durch inverse PCR mit zwei perfekt homologen Primern (1) und (2). Hierzu wird aus der Zellpopulation, die das ausgewählte und zu klonierende Immunglobulin-Gen exprimiert, RNA auf bekannte Weise präpariert. Der zweite Schritt des Prozesses umfaßt zunächst eine enzymatische Umschreibung dieser RNA, z.B. mit Hilfe von Reverser Transkriptase, in cDNA. Die resultierende cDNA wird anschließend durch Ligation in verdünnter Lösung zyklisiert. Infolge der charakteristischen Eigenschaft von Immunglobulin-Genen, bei denen eine konstante Genregion von a priori bekannter Sequenz mit einer variablen Region mit unbekannter Sequenz kombiniert ist, kann nun in einem dritten Schritt mit Hilfe von zwei Primern (1) und (2), die in der konstanten Region hybridisieren, die komplette variable Region ausgehend von diesem zyklischen cDNA-Molekül amplifiziert werden. Dieser Amplifikationsschritt muß nicht notwendigerweise mit Hilfe der als PCR bekanntgewordenen Technik durchgeführt werden. Sämtliche Verfahren zur Amplifikation eines Gensegments mit Hilfe von zwei sequenzspezifischen Primern, die ein klonierbares Nukleinsäure-Fragment der erforderlichen Länge liefern, sind hierzu prinzipiell geeignet. Beispielsweise kommen auch die in EP 88113948.9 oder von Fahy, E., Kwoh, D.Y. und Gingeras, T.R. (1991; PCR Methods and Applications 1, 25-33) beschriebenen Verfahren in Frage. Das auf diese Weise erhaltene erste Amplifizierungsprodukt enthält im allgemeinen den 5'-untranslatierten Bereich des Immunglobulingens, den für das komplette Leaderpeptid kodierenden Bereich, den für die komplette exprimierte V-Region kodierenden Bereich sowie einen Teil des für die C-Region des Immunglobulingens kodierenden Bereich. In Abb. 2 ist dieses erste Amplifizierungsprodukt mit LL'V(D)JΔC bezeichnet.

Die DNA-Fragmente, die komplette V-Regionen der schweren (VH) und der leichten (VL) Kette enthalten, können mit Hilfe von in den verwendeten Primern (1) und (2) vorzugsweise befindlichen Schnittstellen (EcoRI und HindIII) in einen geeigneten Vektor (z.B. pUC18, Yanisch-Perron et al., Gene 33, (1985), 103 - 119) inseriert und sequenziert werden. Alternativ können die amplifizierten Fragmente oder Teile davon auch direkt sequenziert werden.

Mit Hilfe der so erhaltenen Sequenzinformation werden für jede V-Region zwei Oligonukleotid-Primer (3) und (4) entworfen, welche in einem weiteren Prozeßschritt die Amplifikation eines DNA-Fragments mit der in Abb. 2 wiedergegebenen Struktur L'V(D)J gestatten. Dieses Fragment enthält Teile der L'-Region mit einer neu eingeführten Schnittstelle (R), deren Basensequenz das Splicing der RNA unverändert gestattet und nicht zu Aminosäure-Austauschen in L' führt, die die biologische Funktion des Leaderpeptids beeinträchtigen. Derartige geeignete Schnittstellen sind z.B. XhoI (VL) oder CelII (VH). Ein Leichtketten-Vektor,der wie der hier verwendete Vektor ein L'-Aminosäuresegment GlyAlaArgGly aufweist, könnte jedoch anstelle einer Erkennungssequenz für XhoI auch eine oder mehrere Erkennungssequenzen für andere Restriktionsenzyme aufweisen. Aufgrund der Degeneration des genetischen Codes können beispielsweise innerhalb dieses Segments Schnittstellen für ApaI, ApaLI, BssHII, DraIII, NarI, SacII, SmaI oder zahlreiche andere Enzyme eingeführt werden. Analog könnte ein Schwerketten-Vektor mit dem L'- Aminosäuresegment GlyValLeuSer anstelle der CelII-Schnittstelle alternativ mit Schnittstellen für AflII, AhaII, ScaI oder zahlreichen anderen Enzymen innerhalb dieses Segments versehen werden. Weiterhin ist bekannt, daß die Leaderpeptide von Antikörpern funktionell ausgetauscht werden können (Orlandi, R., Güssow, D. H., Jones, P.T. und Winter, G. 1989, Proc. Natl. Acad. Sci. USA 86, 3833 - 3837), so daß alternativ auch andere L'-Segmente (Kabat, E.A., Wu, T.T., Reid-Miller, M., Perry, H.M. und Gottesman, K.S.: Sequences of Proteins of Immunological Interest, 4th edition (1987), U.S. Department of Health and Human Services) zur Verwendung in Frage kämen, die ihrerseits wiederum gleiche oder andere Möglichkeiten zur Einführung von Restriktionsschnittstellen beinhalten. Zu beachten ist lediglich, daß eine Aminosäuresequenz erhalten bleibt, die mit der biologischen Funktion des Leaderpeptids vereinbar ist, und daß die DNA-Sequenz an der Grenze von Intron 1 und L'V(D)J-Exon nicht derart verändert wird, daß ein Splicing des abgelesenen Primärtranskriptes nicht mehr möglich ist (Smith, C.W.J., Porro, E.B., Patton, J.G. und Nadal-Ginard, B. (1989), Nature 342, 243 - 247). Zu diesem Zweck ist es bevorzugt, wenn die aufgrund der Einführung der Schnittstelle resultierende Änderung der DNA-Sequenz nicht zu einer Änderung der Aminosäuresequenz des L'-Leaderpeptidssegments führt. Vorzugsweise enthält der Basisvektor die in das L'-Segment des Leaderpeptids eingeführte Schnittstelle als singuläre Schnittstelle, wodurch die Klonierungsprozedur erheblich erleichtert wird. Die Beseitigung überzähliger Schnittstellen im Vektor kann gegebenenfalls durch bekannte in vitro Mutagenese Verfahren erfolgen.

Das zu amplifizierende Fragment enthält die jeweils vollständige, unveränderte VJ- (VL) bzw. VDJ- (VH) Region des ausgewählten Immunglobulins. Der bei der Amplifizierung verwendete Primer (4) wird vorzugsweise so gewählt, daß das resultierende Amplifizierungsprodukt L'V(D)J an seinem 3'-Ende stumpf mit der letzten Base des Immunglobulin-Exons 2 endet. Diese Amplifikationsprozedur kann ausgehend von der klonierten V-Region oder erneut ausgehend von mRNA bzw. cDNA erfolgen. Anschließend wird das amplifizierte Fragment mit an der in die Sequenz des L'-Leaderpeptidsegments eingefügten Restriktionsstelle (R) mit dem entsprechenden Enzym, z.B. XhoI (VL) oder CelII (VH) geschnitten und in einen Basisvektor gemäß Abb. 1 (Version "c") inseriert, so daß die Struktur eines intakten bzw. operativen Immunglobulin-Genes wieder hergestellt wird.

Die entsprechenden Vektoren verfügen hierzu über entsprechende Schnittstellen, z.B. für XhoI (VL) oder CelII (VH) im L'-Bereich des Leaderpeptids, sowie über Schnittstellen für stumpf schneidende Restriktionsendonukleasen am 3'-Ende des V-Exons, deren 3'-halbseitige Erkennungssequenz genau den drei ersten Basen des Introns 2 von funktionellen Immunglobulingenen entspricht. Da diese Schnittstellen durch die Insertion nicht regeneriert werden müssen, können beliebige V-Exons inseriert werden, solange sie am 3'-Ende stumpf enden. Ein Vergleich der Übergänge von J-Gensegmenten zum Intron 2 bei Immunglobulin-Genen von Maus und Mensch (als beispielhafte Vertreter) zeigt, daß die ersten drei Basen des Introns besonders häufig GTG oder GTA lauten. GTG entspricht der 3'-halbseitigen Erkennungssequenz der stumpf und relativ selten schneidenden Restriktions-Endonuklease BbrPI (CAC/GTG), während GTA in analoger Weise mit der Erkennungssequenz von SnaBI (TAC/GTA) kompatibel ist, welches dieselben Anforderungen erfüllt. Diese Enzyme ermöglichen daher eine Insertion des L'V(D)J-Fragmentes in einen Vektor auf besonders einfache Weise.

Daneben kann dasselbe Ziel jedoch auch erreicht werden, wenn beispielsweise ein Restriktionsenzym verwendet wird, welches einen 3'- oder 5'-Überhang hinterläßt, der mit Exonukleasen entfernt wird, so daß die restliche verbleibende Sequenz der des Introns entspricht. Andere, weniger häufige DNA-Sequenzen am Beginn des Introns eröffnen analoge Möglichkeiten mit anderen Restriktionsenzymen, zumal da ständig neue Endonukleasen beschrieben werden. Eine weitere Möglichkeit zur Erzeugung geeigneter Spaltstellen besteht auch in der Verwendung von Restriktionsenzymen, die außerhalb ihrer Erkennungssequenz schneiden, wie z.B. AlwI oder MnlI, gegebenenfalls unter zusätzlicher Exonuklease-Behandlung. In diesem Fall wird die Erkennungssequenz in denjenigen Teil des Vektors gelegt, der durch das L'V(D)J-Exon ersetzt wird, und zwar in demjenigen, enzymspezifischen Abstand, der eine Öffnung des Vektors genau auf der Exon-Intron-Grenze ermöglicht. Schließlich muß nicht notwendigerweise ein stumpfes PCR-Fragment inseriert werden. DNA-Polymerasen wie beispielsweise die Taq-Polymerase erzeugen Fragmente, an deren Enden zum Teil einzelne Basen, vorzugsweise A, überstehen. Durch Verwendung geeigneter Restriktionsendonukleasen, zum Beispiel HphI, können Vektorenden mit einem hierzu kompatiblen überstehenden T erzeugt werden. Ein derartiges Prinzip ist beispielsweise bei dem Vektor pCR™1000 (Invitrogen Corporation) verwirklicht und könnte in analoger Weise auch bei den hier beschriebenen Vektoren Anwendung finden. Bei der Schnittstelle des Basisvektors am Exon 2/Intron 2 Übergang soll es sich vorzugsweise um eine singuläre Schnittstelle im Basisvektor handeln.

Ein weiterer Gegenstand der vorliegenden Erfindung ist somit ein Basisvektor zur Herstellung eines Expressionsvektors für eine Immunglobulinkette, der folgende essentielle Elemente enthält:
(a) einen zur Expression eines Immunglobulingens geeigneten Promotor,
(b) ein vom Exon 1 eines Immunglobulingens kodiertes Leaderpeptidsegment,
(c) das Intron 1 eines Immunglobulingens,
(d) eine Region, die für den vollständigen C-terminalen Abschnitt eines Immunglobulin-Leaderpeptids oder einen 5'-seitigen Teil davon kodiert,
(e) das komplette Intron 2 eines Immunglobulingens,
(f) eine komplette C-Region eines Immunglobulingens, wobei der Vektor weiterhin folgende Merkmale aufweist:
(g) eine Erkennungssequenz für ein Restriktionsenzym, die derartig lokalisiert ist, daß eine Spaltung des Vektors durch das Restriktionsenzym genau vor dem 5'-Ende von Intron 2 des Immunglobulingens stattfindet, und
(h) eine in der Region (d) eingeführte Erkennungssequenz für ein Restriktionsenzym, wobei die Erkennungssequenz mit der biologischen Funktion der Region (d) vereinbar ist.

Ein weiterer Gegenstand der vorliegenden Erfindung ist ein Basisvektor zur Herstellung eines Expressionsvektors für sowohl die leichte als auch die schwere Immunglobulinkette, der die oben genannten essentiellen Elemente (a) bis (h) jeweils für die leichte Kette eines Immunglobulins als auch für die schwere Kette eines Immunglobulins enthält.

Der zur Expression von Immunglobulingenen vorgesehene Promotor (a) ist vorzugsweise ein in lymphoiden Zellen aktiver Promotor, besonders bevorzugt ein Immunglobulin-Promotor, der frühe oder späte SV40-Promotor, der murine oder humane Cytomegalievirus-Promotor oder der murine oder humane Metallothionin-Promotor. Weiterhin ist bevorzugt, daß der erfindungsgemäße Basisvektor zusätzlich Enhancerelemente enthält, die in lymphoiden Zellen aktiv sind und vorzugsweise aus Immunglobulingenen oder aus Viren, beispielsweise dem Polyoma-, dem SV40-, oder dem murinen oder humanen Cytomegalievirus, stammen.

Die Region (d) aus dem Basisvektor enthält eine Erkennungssequenz für ein Restriktionsenzym, so daß bei Spaltung des Basisvektors mit dem entsprechenden Enzym ein Fragmentende entsteht, an das nach dem erfindungsgemäßen Verfahren ein DNA-Fragment ligiert werden kann, welches den für die komplette exprimierte V-Region kodierenden Sequenzbereich sowie einen 3'-seitigen Teil des L'-Segments enthält.

Im folgenden Beispiel wurden die Erkennungsstellen für die Enzyme SnaBI (g) und XhoI (h) bei einem Leichtketten-Vektor, bzw. für die Enzyme BbrPI (g) und CelII (h) bei einem Schwerkettenvektor verwendet. In den mit XhoI und SnaBI geöffneten Expressionsvektor für die leichte Kette wird ein amplifiziertes und mit SnaBI nachgeschnittenes DNA-Fragment L'VJ (siehe Abb. 2) inseriert. Analog wird mit der schweren Kette unter Verwendung der Enzyme CelII und BbrPI vorgegangen, wobei in den Expressionsvektor für die schwere Kette ein DNA-Fragment L'VDJ (siehe Abb. 2) inseriert wird. Es entstehen zwei Immunglobulin-Gene mit authentischer Struktur, die V-Regionen der gewünschten Spezifität aufweisen. Die Sequenzen der V-Regionen sind in ihren für den sezernierten Antikörper kodierenden Abschnitten gegenüber der natürlichen Aminosäuresequenz völlig unverändert.

Ein weiterer Gegenstand der vorliegenden Erfindung ist ein Verfahren zur Gewinnung von Immunglobulin, welches dadurch gekennzeichnet ist, daß man eine Zelle gleichzeitig oder schrittweise mit 2 kompatiblen Expressionsvektoren transformiert, die gemäß einem oben beschriebenen Verfahren hergestellt worden sind, positiv transformierte Zellen selektioniert, unter geeigneten Bedingungen kultiviert und Immunglobulin aus den Zellen oder dem Kulturmedium gewinnt, wobei einer der Expressionsvektoren ein für die schwere Kette eines ausgewählten Immunglobulins kodierendes Gen enthält und der andere Expressionsvektor ein für die leichte Kette dieses Immunglobulins kodierendes Gen enthält.

Ein weiterer Gegenstand der vorliegenden Erfindung ist ein Verfahren zur Gewinnung von Immunglobulin, welches dadurch gekennzeichnet ist, daß man eine Zelle mit einem Expressionsvektor transformiert, der sowohl ein für die schwere Kette eines ausgewählten Immunglobulins kodierendes Gen als auch ein für die leichte Kette eines ausgewählten Immunglobulins kodierendes Gen enthält und gemäß dem oben beschriebenen Verfahren hergestellt worden ist, positiv transformierte Zellen selektioniert, unter geeigneten Bedingungen kultiviert und Immunglobulin aus den Zellen oder dem Kulturmedium gewinnt.

Die zur Expression der Immunglobuline verwendete Zelle ist vorzugsweise eine lymphoide Säugerzelle, besonders bevorzugt eine "Non-Producer" Hybridomzelle. Beispiele für derartige Zellen sind die Zellinien Sp2/0-Ag 14 (ATCC CRL 1581) und P3X63-Ag 8.653 (ATCC CRL 1580).

Die Transformation der Zelle mit den Vektoren wird vorzugsweise durch eine Elektroporation durchgeführt, es sind jedoch auch andere Transformationsmethoden, wie etwa die Mikroinjektion oder die Lipofektion, einsetzbar.

Bei der Konstruktion der erfindungsgemäßen Vektoren werden vorzugsweise zum Zwecke der besseren Handhabbarkeit alle nicht unmittelbar relevanten DNA-Abschnitte deletiert. Weiterhin ist es bevorzugt, wenn das unter der Kontrolle des SV40-Promoters und -Enhancers exprimierte neo-Gen, welches den transfizierten Säugerzellen Resistenz gegenüber dem Aminoglycosid Neomycin oder Derivaten davon, z.B. G418, verleiht (Southern, P.J. und Berg P. 1981, J.Mol.Appl.Genetics 1, 327 - 341), vom Expressionsvektor für die leichte Kette auf den Expressionsvektor für die schwere Kette transferiert wird. Der SV40-Promoter und -Enhancer kann in diesem Fall auf dem Expressionsvektor der leichten Antikörper-Kette belassen werden. Das neo-Gen wird auf dem Expressionsvektor für die leichte Kette deletiert und auf dem Expressionsvektor für die schwere Kette, z.B. im Austausch für das gpt-Gen, inseriert. Bei der Transfektion von lymphoiden Säugerzellen mit diesen neuen Expressionsvektoren zeigte sich überraschenderweise sowohl ein höherer Anteil Antikörper-sezernierender Zellen unter den G418-resistenten Zellen, als auch eine deutlich erhöhte durchschnittliche Expressionsleistung der erhaltenen Transfectoma-Klone (siehe Beispiel II, letzte Seite). Ein besonders bevorzugter Leichtketten-Vektor zur Herstellung von Expressionsvektoren für beliebige Immunglobuline ist p18023 oder ein Derivat davon und ein besonders bevorzugter Schwerketten-Vektor zur Herstellung von Expressionsvektoren für beliebige Immunglobuline ist p11315 oder ein Derivat davon.

Schließlich betrifft die Erfindung auch ein Verfahren zur Herstellung eines therapeutischen oder/und diagnostischen Mittels, wobei man ein oder mehrere Immunglobuline, die gemäß dem oben beschriebenen Verfahren gewonnen wurden, sowie gegebenenfalls bekannte Hilfs-, Zusatz-, Träger- und Füllstoffe formuliert.

In den nachfolgenden Beispielen wird das erfindungsgemäße Verfahren in Verbindung mit den Zeichnungen und Sequenzprotokollen genauer beschrieben.

Es zeigen:
- Abb. 1:: eine schematische Darstellung verschiedener Klonierungsvarianten zur Erzeugung von Immunglobulin-Expressionsvektoren,
- Abb. 2:: eine schematische Darstellung der erfindungsgemäßen Gewinnung von DNA-Fragmenten, die zur Klonierung in einen Basisvektor verwendet werden,
- Abb. 3:: eine schematische Darstellung des Leichtkettenvektors p15044,
- Abb. 4:: eine schematische Darstellung der Konstruktion des Leichtkettenvektors p18023,
- Abb. 5:: eine schematische Darstellung des Leichtketten-Expressionsvektors p16031,
- Abb. 6:: eine schematische Darstellung der Konstruktion des Schwerkettenvektors p15045,
- Abb. 7:: eine schematische Darstellung der Konstruktion des Schwerkettenvektors p11315,
- Abb. 8:: eine schematische Darstellung des Schwerketten-Expressionsvektors p16026,
- SEQ ID NO. 1:: die V-Region der L-Kette des MAK A23A41, wobei die Aminosäuren -15 bis -1 das Signalpeptid (unvollständig infolge der Art der Klonierung), die Aminosäuren 1-96 die V-Region, die Aminosäuren 97-107 die J-Region und die Aminosäuren 108-118 den Beginn der konstanten Region umfassen,
- SEQ ID NO. 2:: die V-Region der H-Kette des MAK A23A41, wobei die Aminosäuren -19 bis -1 das Signalpeptid, die Aminosäuren 1-98 die V-Region, die Aminosäuren 99-104 die D-Region, die Aminosäuren 105-118 die J-Region und die Aminosäuren 119-131 den Beginn der konstanten Region umfassen,
- SEQ ID NO. 3:: den für die Synthese eines LL'VJΔC-Fragments aus A23A41 verwendeten Kappa I-Primer,
- SEQ ID NO. 4:: den für die Synthese eines LL'VJΔC-Fragments aus A23A41 verwendeten Kappa II-Primer,
- SEQ ID NO. 5:: den für die Synthese eines LL'VDJΔC-Fragments aus A23A41 verwendeten γ1I-Primer,
- SEQ ID NO. 6:: den für die Synthese eines LL'VDJΔC-Fragments aus A23A41 verwendeten γ1II-Primer,
- SEQ ID NO. 7:: den für die Einführung einer XhoI-Stelle in p11225 verwendeten 5'-Primer,
- SEQ ID NO. 8:: den für die Einführung einer XhoI-Stelle in P11225 verwendeten 3'-Primer,
- SEQ ID NO. 9:: den für die Einführung einer SnaBI-Stelle in p11225 verwendeten 5'-Primer,
- SEQ ID NO. 10:: den für die Einführung einer SnaBI-Stelle in p11225 verwendeten 3'-Primer,
- SEQ ID NO. 11:: das BglII/NotI-Fragment aus p15044,
- SEQ ID NO. 12:: den für die Synthese eines L'VJ-Fragments aus A23A41 verwendeten LI-Primer,
- SEQ ID NO. 13:: den für die Synthese eines L'VJ-Fragments aus A23A41 verwendeten LII-Primer,
- SEQ ID NO. 14:: das für die Einführung einer BbrPI-Schnitt stelle in den Vektor p11221 verwendete Oligonukleotid,
- SEQ ID NO. 15 und SEQ ID NO. 16:: das synthetische EcoRI/Asp700-Fragment zur Ein führung einer CelII-Schnittstelle in den Vektor p15041,
- SEQ ID NO. 17:: das NotI-Fragment aus P15045,
- SEQ ID NO. 18 und SEQ ID NO. 19:: den für die Einführung einer EcoRV-Schnitt stelle in den Vektor p11201 verwendeten Adapter,
- SEQ ID NO. 20:: den für die Synthese eines L'VDJ-Fragments aus A23A41 verwendeten Primer HI und
- SEQ ID NO. 21:: den für die Synthese eines L'VDJ-Fragments aus A23A41 verwendeten Primer HII.

In dieser Erfindung genannte Plasmide und Zellinien wurden bei der Deutschen Sammlung für Mikroorganismen und Zellkulturen GmbH (DSM), Mascheroder Weg 18, D-3300 Braunschweig und bei der American Type Culture Collection (ATCC), 12301 Parklawn Drive, Rockville, Maryland, unter den folgenden Hinterlegungsnummern hinterlegt:
- Hybridoma-Zellinie A23A41: DSM ACC 2015
- Vektor p11225: DSM 5094
- Vektor p11224: DSM 5093
- Hybridoma-Zellinie Sp2/0Ag14: ATCC CRL 1581.

### BEISPIEL I:

### KLONIERUNG VON ANTIKÖRPER-V-REGIONEN

Die murine Hybridoma-Linie A23A41 sezerniert einen Antikörper gegen die β-Kette des humanen Interleukin2-Rezeptors. Dieser Antikörper besteht aus einer schweren Kette vom Isotyp γ1 und einer leichten Kette vom Isotyp kappa.

Von der murinen Hybridoma-Linie A23A41 (DSM ACC 2015) werden 10⁶ Zellen in einem Eppendorf-Reaktionsgefäß mit 500 µl Lyse-Puffer (1,272 g LiCl, 3,963 g Harnstoff, 66,6 µl 3 mol/l Natriumacetat pH 5,2, 200 µl 10 % SDS, 2 mg Heparin, H₂O ad 10 ml) versetzt. Zur Fragmentierung chromosomaler DNA wird die Lösung mehrfach durch eine Kanüle passagiert. Die Lösung wird über Nacht bei 4°C zur Präzipitation der RNA inkubiert.

Anschließend wird das Präzipitat abzentrifugiert und zweimal in 235 µl Waschpuffer (1,696 g LiCl, 5,285 g Harnstoff, 2 mg Heparin, 66,6 µl 3 mol/l Natriumacetat pH 5,2, H₂O ad 10 ml) gewaschen. Das Präzipitat wird dann in 200 µl 0,3 mol/l Natriumacetat pH 5,2 /0,1 % SDS gelöst und zweimal mit einem Volumen Phenol (äquilibriert in 3 % NaCl) extrahiert. Die RNA wird aus der wässrigen Phase durch Zusatz von 0,5 ml Ethanol über Nacht bei 4°C präzipitiert. Typischerweise werden so ca. 2 µg RNA gewonnen.

100 - 200 ng RNA werden unter Verwendung eines kommerziell erhältlichen cDNA-Synthese-Kits (Boehringer Mannheim GmbH, Katalog-Nr. 1117 831) in doppelsträngige cDNA umgeschrieben. Die cDNA wird mit Ethanol präzipitiert und anschließend in 10 µl H₂O aufgenommen. Zur Bildung zyklischer cDNA-Moleküle wird T4-Ligase und entsprechender Puffer zugesetzt (Sambrook, J., Fritsch, E.F., Maniatis, T.: Molecular cloning - a laboratory manual, second edition, Cold Spring Harbor Laboratory Press, Cold Spring Harbor, New York 1989) und über Nacht bei 16°C inkubiert. Anschließend wird T4-Ligase durch eine Inkubation von 10 Minuten bei 65°C inaktivert.

Je ein Drittel dieses Ligationsansatzes (entsprechend 30 - 60 ng eingesetzter RNA) wird für eine PCR-Reaktion mit je 100 pmol der Primer Kappa I (1) und Kappa II (2) für die leichte Kette, beziehungsweise Gamma1 I (1) und Gamma1 II (2) für die schwere Kette eingesetzt. Die Primer-Sequenzen wurden aus den konstanten Immunglobulin-Gensegmenten der entsprechenden Antikörper-Subklasse Kappa bzw. Gamma1 abgeleitet, die in öffentlich zugänglichen Sequenzdatenbanken verfügbar sind.

### Kappa I:

5'CCCCGAATTC GAGGCTTCCC CACAAGCGAC CTACCACTGT T 3' (SEQ ID NO. 3)

### Kappa II:

5'CCCCAAGCTT GGAAGATGGA TACAGTTGGT GCAGCATCAG C 3' (SEQ ID NO. 4)

### Gamma1 I:

5'GGGGGAATTC CATACTGAGA AGAGCCTCTC CCACTCTCCT G 3' (SEQ ID NO. 5)

### Gamma1 II:

5'GGGGAAGCTT GGGGCCAGTG GATAGACAGA TGGGGGTGTC G 3' (SEQ ID NO. 6)
Die übrigen Reagenzien für die PCR werden dem GeneAmp^{tm} DNA Amplification Reagent Kit (Perkin Elmer, Best.Nr. N801-0055) entnommen und wie dort beschrieben eingesetzt. Es werden 30 Zyklen mit folgenden Inkubationszeiten durchgeführt: 1 Minute 94°C, 2 Minuten 50°C, 3 Minuten 72°C. Die Temperaturübergänge finden während jeweils einer Minute statt.

Ein Aliquot jedes PCR-Ansatzes (typischerweise 10 - 50 µl) wird mit EcoRI und HindIII restringiert und auf einem Agarose-Gel aufgetrennt. Das DNA-Fragment der erwarteten Größe wird nach etablierten Methoden aus dem Gel eluiert und in übliche Klonierungsvektoren zur weiteren Charakterisierung und Sequenzierung inseriert. Die auf diese Weise erhaltene DNA-Sequenz der V-Regionen des Antikörpers A23A41 ist in den Sequenzprotokollen SEQ ID NO. 1 und SEQ 1D NO. 2 gezeigt. Ein definiertes, auf einem Gel identifizierbares DNA-Fragment muß nicht in allen Fällen auftreten, da auch partiell degradierte RNA amplifizierbare cDNA liefert, so daß auch verkürzte Fragmente auftreten können. In diesen seltenen Fällen wird DNA aus dem gesuchten Größenbereich kloniert und nach etablierten Methoden sequenziert. Eine weitere alternative Vorgehensweise stellt die direkte Sequenzierung der amplifizierten DNA dar.

Als Produkt der hier beschriebenen Klonierung wird die komplette Antikörper-V-Region in einem Fragment LL'V(D)JΔC erhalten. Für die Expression einer unveränderten V-Region wird daraus gemäß dem folgenden Beispiel das L'V(D)J-Fragment mittels zweier aus der Sequenz abgeleiteter Primer (3) und (4) über PCR erhalten und in geeignete Expressionsvektoren kloniert.

### BEISPIEL II:

### EXPRESSION REKOMBINANTER ANTIKÖRPER

### II.1 KONSTRUKTION DER EXPRESSIONSVEKTOREN

Ausgangsmaterial zur Konstruktion dieser Expressionsvektoren sind die in EP-A 0 378 175 beschriebenen Vektoren, die humane konstante Regionen vom Typus Kappa und γ1 enthalten. Entsprechend ermöglichen sie je nach Herkunft der verwendeten V-Regionen die Expression chimärer oder humaner Antikörper.

### II.1.1. Expressionsvektoren für die leichte Kette

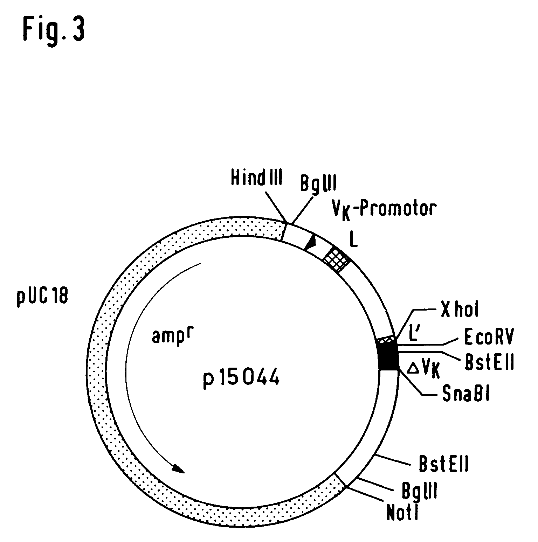

### a) Konstruktion von p15044 (Abb. 3)

Als Ausgangsvektor wurde der Expressionsvektor für die leichte Kette des chimären Antikörpers MAK〈TSH〉, p11225, verwendet; p11225 ist in EP-A 0 378 175 beschrieben und bei der DSM unter der Nummer DSM 5094 hinterlegt. In diesen Vektor wurden mit Hilfe einer PCR-Mutagenese die Schnittstellen der Enzyme XhoI und SnaBI eingebracht. Die beiden Spaltstellen wurden in zwei getrennten PCR-Reaktionen eingeführt und die amplifizierten Fragmente anschließend unter Deletion der homologen Bereiche in ein Derivat von p11225 kloniert, welches an Immunglobulin- Gensegementen lediglich das 1023 bp BglII/NotI-Fragment von p11225, kloniert in pUC18, enthielt. Auf diesem 1023 bp großen BglII/NotI-Fragment liegen Promoter, L- und L'VJ-Exon (Dieser Zwischenschritt war erforderlich, da p11225 zu viele Spaltstellen für die verwendeten Restriktionsenzyme enthält). Das bei der Insertion der amplifizierten Fragmente mit den XhoI und SnaBI-Schnittstellen in das Derivat von p11225 resultierende Plasmid wurde als p15044 bezeichnet (Abb. 3). Die Sequenz des veränderten BglII/NotI-Fragments ist im Sequenzprotokoll SEQ.ID.NO 11 dargestellt.

Die PCR-Reaktion zur Einführung der XhoI-Schnittstelle wurde mit einem 5'-Primer durchgeführt, der ohne Fehlpaarungen oberhalb der Promoter-Region hybridisierte, und mit einem 3'-Primer, der die neue Schnittstelle in den L'-Anteil des V-Exons einbrachte. Im Falle der die SnaBI-Schnittstelle einführenden PCR-Mutagenese enthielt der 5'-Primer die neue Schnittstelle und zerstörte gleichzeitig die hier liegende interne XhoI-Erkennungssequenz. Der 3'-Primer war zu einer Sequenz 200 bp stromabwärts aus dem Intron 2 perfekt komplementär. Die verwendeten Primer besaßen folgende Sequenz:

### XhoI, 5'-Primer:

5'TGCCAAGCTT GCATGCCTGC AGGTCGAGAT CT 3' (SEQ ID NO. 7) (fettgedruckt die Schnittstellen HindIII und BglII)

XhoI, 3'-Primer:
5'CACGATATCA CCTCGAGCAC CTGGAAAATT AGGAAAAAAA AAAGTTTAGT
AGGACAC 3'    (SEQ ID NO. 8)
(fettgedruckt die Schnittstellen für EcoRV und XhoI)

### SnaBI, 5'-Primer:

5'CCCGGTTACC GAGCTGATAC GTAAGTACAC 3' (SEQ ID NO. 9) (fettgedruckt die Schnittstellen für BstEII und SnaBI)

### SnaBI, 3'-Primer:

5'TCTCTTGGAT GGTGACCATA G 3' (SEQ ID NO. 10) (fettgedruckt die Schnittstelle für BstEII)

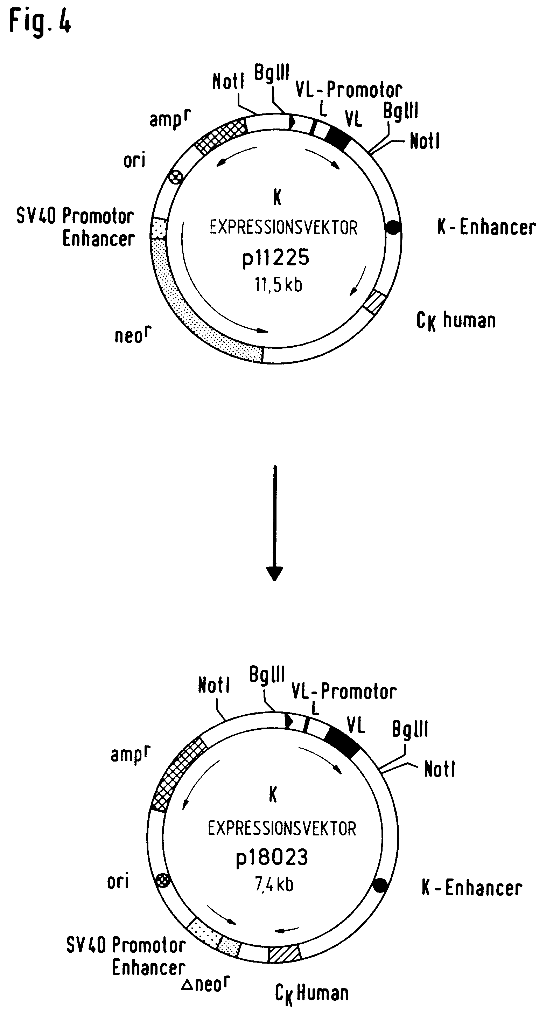

### b) Konstruktion von p18023 (Abb.4)

Das Plasmid p18023 wurde von dem Leichtketten-Expressionsvektor p11225 abgeleitet mit dem Ziel, das neo-Gen und alle funktionslosen DNA-Abschnitte dieses Vektors zu deletieren. Zur Konstruktion von p18023 wurde p11225 (beschrieben in EP-A 0 378 175) mit dem Enzym SauI partiell gespalten und das 2,8 kb große Fragment entfernt, welches im wesentlichen Sequenzen enthält, die sich 3' an das humane C-kappa-Exon anschließen. Der Vektor wurde daraufhin mit E.coli DNA- Polymerase I, Klenow-Fragment, behandelt und ein SalI-Linker (New England Biolabs, Kat.Nr. 1027) eingefügt. Anschließend wurde durch Restriktion mit den Enzymen Sfil und SalI ein Großteil des neo-Gens entfernt, welches dadurch seine Funktionalität verliert. Die Schnittstellen wurden mit T4- Polymerase aufgefüllt und ligiert, wobei man den Vektor p18023 erhält.

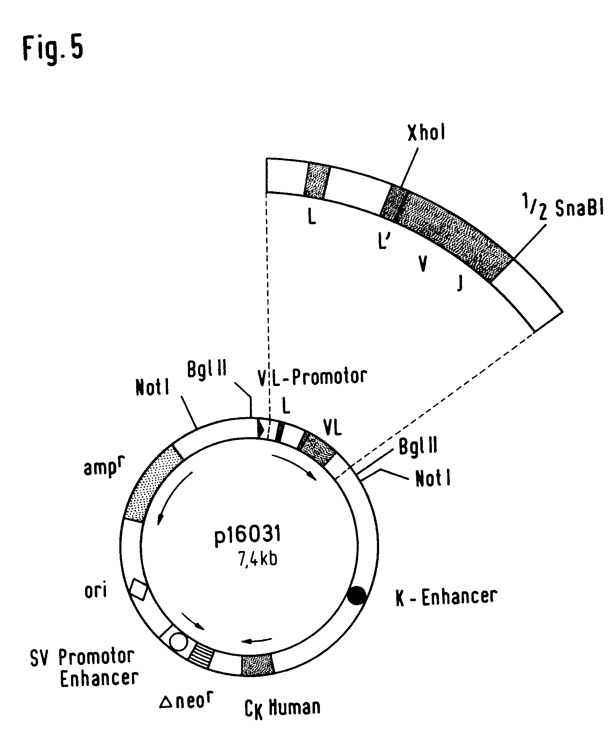

### c) Konstruktion von p16031 (Abb. 5)

Anhand der Nukleotid-Sequenz der leichten Kette von MAK A23A41, die wie in Beispiel I beschrieben erhalten wurde, wurden zwei sequenzspezifische Oligonukleotide LI und LII entworfen. Mit Hilfe der Oligonukleotide
LI (5'AGCCTCTCGA GGTGACGTCT TGC 3') (SEQ ID NO. 12) und
LII (5'GTTTAATTTC CAGCTTGGTC 3') (SEQ ID NO. 13)
wurde aus mRNA der Hybridomlinie A23A41 das dem L'VJ-Exon entsprechende Fragment amplifiziert (die Oligonukleotide LI und LII hybridisieren mit der zu Nukleotid 113-135 von SEQ.ID.NO 1 komplementären Sequenz bzw. mit Nukleotid 426-447 von SEQ.ID.NO 1). Dieses Fragment wurde mit T4-Polymerase behandelt, anschließend mit XhoI nachgeschnitten und in zuvor mit XhoI und SnaBI geschnittenes p15044 ligiert. Aus dem resultierenden Plasmid wurde das BglII-Fragment, welches Promoter, L- und L'VJ- Exon enthält, entnommen und anstelle des homologen Fragments in p18023 ligiert. Das resultierende Plasmid p16031 ist in Abb. 5 dargestellt. Es ermöglicht die Expression einer chimären kappa-Kette mit der Spezifität von MAK A23A41.

Zu Vergleichszwecken wurde das BglII-Fragment auch anstelle des homologen BglII-Fragmentes in p11225 (DSM 5094) inseriert (p11225-AA1). Dieser Vektor enthält im Gegensatz zu p16031 noch ein vollständiges neo-Gen, sowie die Sequenzen 3' von C-kappa.

### II.1.2. Expressionsvektoren für die schwere Kette

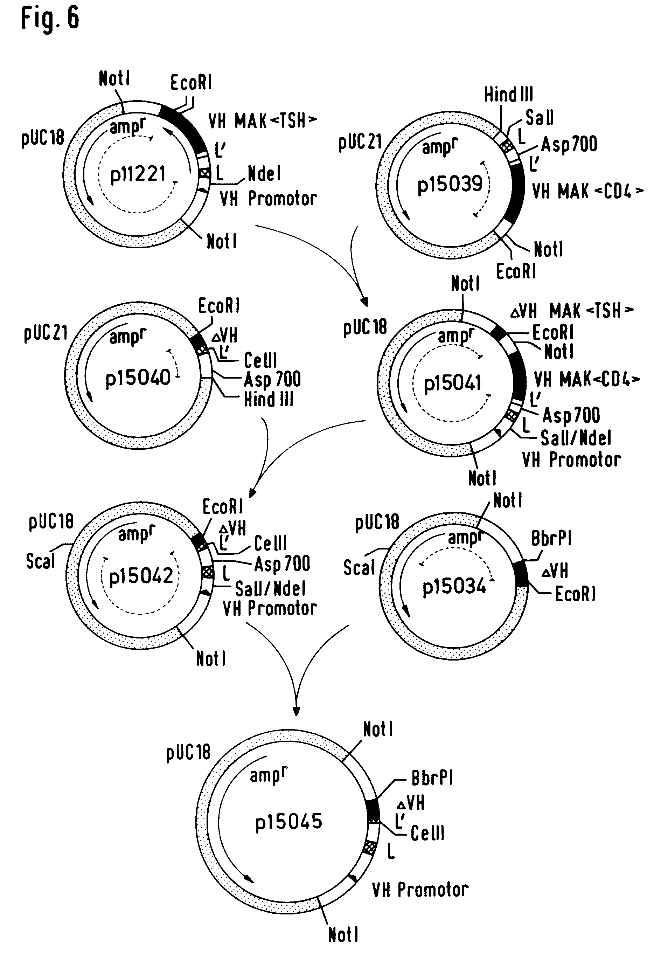

### a) Konstruktion von p15045 (Abb. 6)

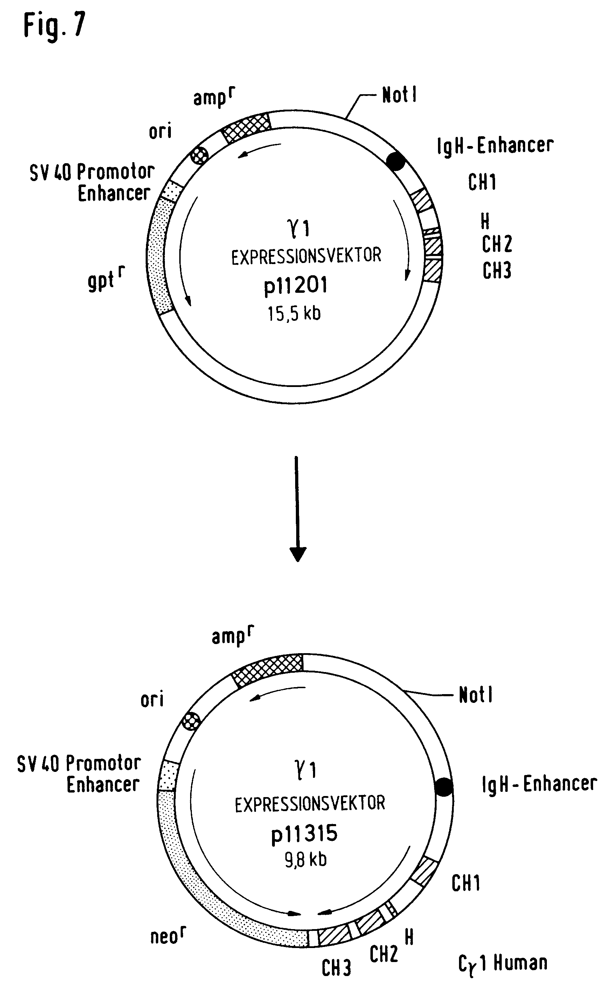

Hierzu wurde zunächst die BbrPI-Schnittstelle am 3'-Übergang des VDJ-Exons zum Intron 2 in p11221, einem verkürzten Derivat von p11224 (beide beschrieben in EP-A 0 378 175), durch Oligonukleotid-gestützte Mutagenese (Inouye, S. und Inouye, M. (1987), Synth. Appl. DNA RNA (Hrsg. A.Narang), Academic Press 181 - 206) unter Verwendung des Oligonukleotides
5'TCACTGTCTC TGCACGTGAG TCCTAACTTC 3' (SEQ ID NO. 14)
eingeführt (p15034, Abb. 6). Die weitere Konstruktion von p15045 erfolgte in der in Abb. 6 dargestellten Klonierungs-Kaskade, wobei als Ausgangsmaterial neben p11221 auch ein genomisches Fragment der funktionell rearrangierten Schwerkette der Hybridomlinie MAK 〈CD4〉 MT15.1 verwendet wurde. Die Klonierung dieses genomischen Fragments ist beschrieben in EP-A 0 481 502. Von diesem in pUC21 subklonierten Fragment (ergibt p15039) wurde ein SalI (in der 5'-untranslatierten Region)-EcoRI (im Intron 2) Fragment abgespalten. Dieses 470 bp Fragment enthielt L, Intron 1, L'VDJ und einen kleinen Teil Intron 2. Außerdem verfügte es im Gegensatz zur Sequenz aus p11221 über eine Asp700 Schnittstelle in Intron 1, die für die weitere Klonierung wichtig war. Zunächst wurde p11221 mit NdeI (in der 5' untranslatierten Region) geöffnet, mit DNA-Polymerase, Klenow-Fragment, behandelt und anschließend mit EcoRI (in V) das 470 bp Fragment deletiert, welches L'VDJ- und L-Exon enthält. Anschließend wurde das homologe SalI-EcoRI Fragment (SalI mit Klenow behandelt) aus MAK 〈CD4〉 MT15.1 inseriert und damit die für die weitere Klonierung notwendige Asp700 Schnittstelle im Intron 1 eingeführt. Das resultierende Plasmid p15041 wurde mit EcoRI und Asp700 geöffnet und mit einem synthetischen EcoRI Asp700 Fragment ligiert, welches aus den Oligonukleotiden 
5'CCCCAAGCTT GAAGCAGTTC CATGAATACT CACCTTCCTG TGTTCTTTTC
ACAGGTGTGC TCAGCGAGGT CCAGCTGGAA TTCCC 3'  (SEQ ID NO. 15)
und
5'GGGAATTCCA GCTGGACCTC GCTGAGCACA CCTGTGAAAA GAACACAGGA
AGGTGAGTAT TCATGGAACT GCTTCAAGCT TGGGG 3'  (SEQ ID NO. 16)
bestand, und über das die gewünschte CelII Schnittstelle im L'-Bereich eingeführt wurde. Das synthetische Fragment war zuvor in pUC21 kloniert worden (als p15040). Das bei der Insertion des synthetischen EcoRI/Asp700-Fragments in p15041 resultierende Plasmid p15042 enthielt somit bereits die gewünschte Genstruktur aus Immunglobulin-Promoter, Leader-Exon und einem Teil des L'VDJ-Exons mit der Schnittstelle für CelII. Dieses Plasmid p15042 wurde nun mit ScaI und EcoRI geöffnet und mit dem ScaI EcoRI Fragment aus p15034 (Abb. 7) ligiert, welches die gewünschte BbrPI Schnittstelle am 3'-Ende des Exons 2 enthält. Das resultierende Plasmid p15045 ist in Abb. 6 schematisch dargestellt. Im Sequenzprotokoll SEQ.ID.NO 17 ist die Sequenz des veränderten NotI-Fragments angegeben.

### b) Konstruktion von p11315 (Abb. 7)

Das Plasmid p11315 wurde von dem Schwerketten-Expressionsvektor p11224 (EP-A 0 378 175, DSM 5093) abgeleitet mit dem Ziel, einerseits das gpt-Gen (kodierend für Resistenz gegenüber Mycophenolsäure) gegen ein neo-Gen (kodierend für G418-Resistenz) auszutauschen, und andererseits nicht-relevante DNA-Bereiche zu deletieren.
Hierfür wurde das Plasmid p11201 (beschrieben in EP-A 0 378 175, erhältlich aus p11224 durch Deletion eines ca. 1 kb großen NotI-Fragments, das die für die V-Region kodierende Sequenz enthält) verwendet, welches die für die konstante Region der humanen γ1 Kette codierenden Sequenzen enthält. Das Plasmid p11201 wurde mit dem Restriktionsenzym NdeI zwischen dem bakteriellen Replikationsursprung und dem Mycophenolsäure-Resistenzgen gespalten. Die NdeI-Schnittstelle wurde mit dem doppelsträngigen Adapter, der aus 2 komplementären Oligonukleotiden
5'GGGATATCAC GCGTACGGAT CCAACA 3' (SEQ ID NO. 18) und
5'TATGTTGGAT CCGTACGCGT CATATCCC 3' (SEQ ID NO. 19)
zusammengesetzt war, ligiert, durch den nun unmittelbar neben der NdeI Schnittstelle eine EcoRV Schnittstelle zur Verfügung stand. Anschließend wurde mit EcoRV und SfiI nachgeschnitten. Hierdurch wurde ein 8,5 kb großes Fragment aus p11201 deletiert, welches das gpt-Gen und die nicht relevanten humanen DNA-Sequenzen enthält, die sich 3' an die Cγ1-Region anschließen. Allerdings wurde hierdurch auch das Exon für CH3 des humanen γ1-Gens deletiert. Dieses wurde durch Insertion des entsprechenden 551 bp großen SfiI-StuI Fragmentes aus p11201 wieder ergänzt. Schließlich wurde das Neomycin-Resistenzgen dem Plasmid pSV2neo (Southern, P.J. und Berg P. 1981, J.Mol.Appl.Genetics 1, 327 - 341) als 2,9 kb großes Fragment der Enzyme NdeI und BamHI entnommen und in den mit den gleichen Enzymen geöffneten, verkürzten Expressionsvektor inseriert. Das resultierende Plasmid wurde als p11315 (Abb. 7) bezeichnet.

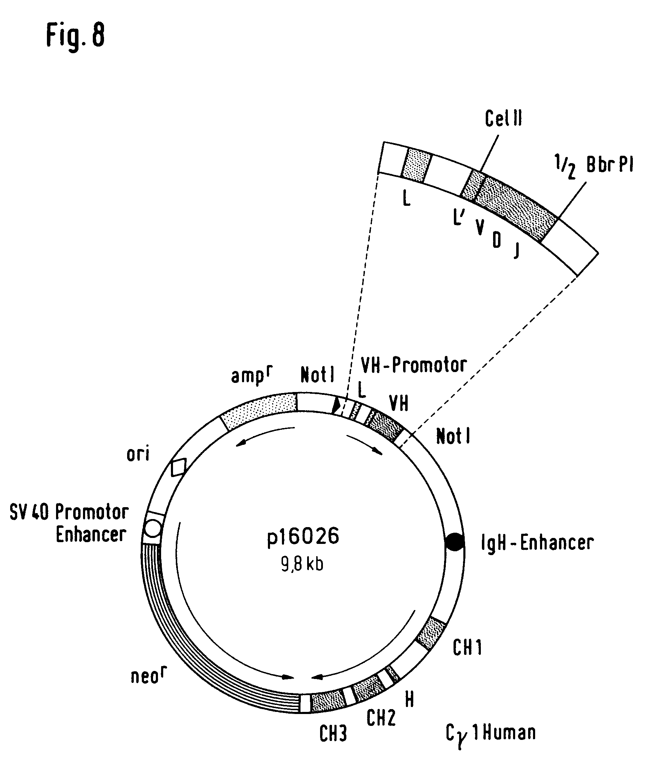

### c) Konstruktion von p16026 (Abb. 8)

Anhand der Nukleotid-Sequenz der schweren Kette von MAK A23A41, die wie in Beispiel I beschrieben erhalten wurde, wurden zwei sequenz-spezifische Oligonukleotide HI und HII entworfen. Mit Hilfe der Oligonukleotide
HI (5'TGCAGGTGTG CTCAGCGAGG TCCAGCTG 3') (SEQ ID NO. 20) und
HII (5'CTGAGGAGAC GGTGACTGAG 3') (SEQ ID NO. 21)
wurde aus der mRNA der Hybridomlinie A23A41 das dem L'VDJ-Exon entsprechende Fragment amplifiziert (die Oligonukleotide HI und HII hybridisieren mit der zu Nukleotid 175-202 von SEQ.ID.NO. 2 komplementären Sequenz bzw. mit Nukleotid 526-545 von SEQ.ID.NO.2). Das erhaltene Fragment wurde mit T4-Polymerase behandelt, mit CelII nachgeschnitten und in das mit CelII und BbrPI geöffnete Plasmid p15045 inseriert. Anschließend wurde das NotI-Fragment aus dem resultierenden Plasmid, welches Immunglobulin-Promoter, L- und L'VDJ-Exon enthielt, in die einzige NotI-Schnittstelle von p11315 ligiert, und zwar so, daß die Expressionsrichtung aller Immunglobulin-Gensegmente gleich war. Das resultierende Plasmid p16026 ist in Abb. 8 dargestellt. Es ermöglicht die Expression einer chimären Antikörper-Kette mit humanen konstanten Regionen vom Typus γ1, deren V-Region die Spezifität von MAK A23A41 besitzt.

Zu Vergleichszwecken wurde das NotI-Fragment auch anstelle des homologen NotI-Fragmentes in p11224 (DSM 5093) inseriert (p11224-A421). Dieser Expressionsvektor enthält im Gegensatz zu p16026 noch das gpt-Gen anstelle des neo-Gens.

### II.2: TRANSFEKTION VON SÄUGERZELLEN MIT DEN EXPRESSIONSPLASMIDEN

Sowohl Vektor p16031 als auch Vektor p16026 wurden an ihrer einzigen PvuI-Schnittstelle (im bakteriellen amp^{R}-Gen) linearisiert. Sp2/0 Ag14-Zellen (ATCC CRL1581) wurden auf RPMI-1640 Medium (G.E.Moore, R.E.Gerner & H.A.Franklin (1967) Culture of normal Human Leukocytes. J.Am.Med.Assoc. 199, 519-526) angezogen, das mit 10 % fötalem Kälberserum, 20 mmol/l Glutamin, 1 mmol/l NaPyruvat und Aminosäuren (MEM Aminosäuren, Zusammensetzung vergl. R.G.Ham (1963), Exp. Cell. Res. 29, 515- 526) supplementiert war. Exponentiell wachsende Zellen (ca. 5 x 10⁵ /ml) wurden ad 1,25 x 10⁶/ml in 1 x HeBS-Puffer (G.Chu et al. (1987), Nucl. Acids Res. 15, 1311- 1326) resuspendiert. Linearisierter Vektor p16031 und p16026 wurde in einer Konzentration von je 12,5 µg/ml zugegeben. Zellen und DNA wurden für 10 Minuten bei 0 °C vorinkubiert, anschließend in 800 µl Aliquoten einem einmaligen elektrischen Puls von 230 V (Bio Rad Gene Pulser, bei 960 µF) ausgesetzt und danach weitere 10 Minuten bei 0 °C inkubiert. Daraufhin wurden die Zellen in RPMI- Medium (mit den beschriebenen Zusätzen) auf 96-er Mikrotiterplatten in geeigneten Verdünnungen abgelegt.

24 h nach Transfektion wurde mit G418 (Geneticin Gibco, 1 mg/ml) selektiert. Nach etwa 2 Wochen wurden die herangewachsenen Transfectoma-Klone mit Hilfe eines geeigneten ELISA-Nachweises auf Produktion von chimären Antikörpern untersucht.

In einem typischen Experiment ergaben sich folgende durchschnittliche Expressionsleistungen für chimären MAK A23A41 bei Verwendung der Vektoren p16026 und p16031 einerseits und p11225-A41 und p11224-A41 andererseits:
p11225-A41/p11224-A41: 18 % der G418-resistenten Transfectoma-Klone bilden Antikörper mit einer durchschnittlichen Expressionsleistung von 250 ng/ml x 10⁶ Zellen in 24 h (Standardabweichung 260 ng/ml).

p16031/p16026: 48 % der G418-resistenten Transfectoma-Klone bilden Antikörper mit einer durchschnittlichen Expressionsleistung von 500 ng/ml x 10⁶ Zellen in 24 h (Standardabweichung 400 ng/ml).

Die Verwendung von p16026 und p16031 führt also überraschenderweise zu einer signifikanten Verbesserung sowohl der Klonausbeute als auch der Expressionsleistung.

SEQUENCE LISTING

(2) INFORMATION ZUR SEQ ID NO:1:

(i) SEQUENZCHARAKTERISTIKEN:
(A) LÄNGE: 486 Basenpaare
(B) TYP: Nukleinsäure
(C) STRANGFORM: Einzelstrang
(D) TOPOLOGIE: linear

(ii) MOLEKÜLTYP: cDNA zu mRNA

(ix) MERKMAL:
(A) NAME/BEZEICHNUNG: kodierende Sequenz
(B) POSITION: 81..479

(ix) MERKMAL:
(A) NAME/BEZEICHNUNG: reifes Peptid
(B) POSITION: 127..479

(ix) MERKMAL:
(A) NAME/BEZEICHNUNG: Signalpeptid
(B) POSITION: 81..126

(xi) SEQUENZDARSTELLUNG: SEQ ID NO:1:

GAATTCGAGG CTTCCCCACA AGCGACCTAC CACTGTTGCG GTGCTCCAAA CYTCCTCCCC 60

ACCTCCTTTT CCTCCTCCTC CCT TTC CTT GGC TTT TGS CTT TYT TGG ATT 110
Pro Phe Leu Gly Phe Xaa Leu Xaa Trp Ile
-15 -10

CCA GCC TCC AGA GGT GAC GTC TTG CTG ACT CAG TCT CCA GCC ATC CTG 158
Pro Ala Ser Arg Gly Asp Val Leu Leu Thr Gln Ser Pro Ala Ile Leu
-5 1 5 10

TCC GTG AGT CCA GGA GAA AGA GTC AGT TTC TCC TGT AGG GCC AGT CAG 206
Ser Val Ser Pro Gly Glu Arg Val Ser Phe Ser Cys Arg Ala Ser Gln
15 20 25

AGC ATT GGC ACA AGC ATA CAC TGG TAT CAG CAA AGA ACA AAT GGT CCT 254
Ser Ile Gly Thr Ser Ile His Trp Tyr Gln Gln Arg Thr Asn Gly Pro
30 35 40

CCA AGG CTT CTC ATA AAG TAT GCG TCT GAG TCA ATC TCT GGG ATC CCT 302
Pro Arg Leu Leu Ile Lys Tyr Ala Ser Glu Ser Ile Ser Gly Ile Pro
45 50 55

TCC AGG TTT AGT GGC AGT GGA TCA GGG ACA GAT TTT ACT CTT AGC ATC 350
Ser Arg Phe Ser Gly Ser Gly Ser Gly Thr Asp Phe Thr Leu Ser Ile
60 65 70 75

AGC AGT GTG GAG TCT GAA GAT ATT GCA GAT TAT TAC TGT CAA CAA ACT 398
Ser Ser Val Glu Ser Glu Asp Ile Ala Asp Tyr Tyr Cys Gln Gln Thr
80 85 90

AAT AGC TGG CCA ACC ACG TTC GGA GGG GGG ACC AAG CTG GAA ATT AAA 446
Asn Ser Trp Pro Thr Thr Phe Gly Gly Gly Thr Lys Leu Glu Ile Lys
95 100 105

CGG GCT GAT GCT GCA CCA ACT GTA TCC ATC TTC CAAGCTT 486
Arg Ala Asp Ala Ala Pro Thr Val Ser Ile Phe
110 115

(2) INFORMATION ZU SEQ ID NO:2:

(i) SEQUENZCHARAKTERISTIKEN:
(A) LÄNGE: 588 Basenpaare
(B) TYP: Nukleinsäure
(C) STRANGFORM: Einzelstrang
(D) TOPOLOGIE: linear

(ii) MOLEKÜLTYP: cDNA zu mRNA

(ix) MERKMAL:
(A) NAME/BEZEICHNUNG: kodierende Sequenz
(B) POSITION: 134..583

(ix) MERKMAL:
(A) NAME/BEZEICHNUNG: reifes Peptid
(B) POSITION: 192..583

(ix) MERKMAL:
(A) NAME/BEZEICHNUNG: Signalpeptid
(B) POSITION: 134..191

(xi) SEQUENZDARSTELLUNG: SEQ ID NO:2:

GAATTCCATA CTGAGAAGAG CCTCTCCCAC TCTCCTGGTA AATRATCCCA GTGTCCTTGG 60

AGCCCTCTGG TCCTACAGGA CTCTGACACC TACCTCCACC CCTCCACAGA CACTGAACAC 120

ACTGACTCTA ACC ATG GAA TGG AGC TGG ATC TTT CTC TTT CTC CTG TCA 169
Met Glu Trp Ser Trp Ile Phe Leu Phe Leu Leu Ser
-19 -15 -10

GGA ACT GCA GGT GTC CTC TCT GAG GTC CAG CTG CAA CAG TTT GGA GCT 217
Gly Thr Ala Gly Val Leu Ser Glu Val Gln Leu Gln Gln Phe Gly Ala
-5 1 5

GAA TTG GTG AAG CCT GGG ACT TCG GTG AAG ATA TCC TGC AAG GCT TCT 265
Glu Leu Val Lys Pro Gly Thr Ser Val Lys Ile Ser Cys Lys Ala Ser
10 15 20 25

GGC TAC ATT TTC ACT GAC TAC AAC ATG GAC TGG GTG AAG CAG AGC CAT 313
Gly Tyr Ile Phe Thr Asp Tyr Asn Met Asp Trp Val Lys Gln Ser His
30 35 40

GGA AAG AGC CTT GAG TGG ATT GGA GAT ATT GAT CCT AAC TTT GAT AGT 361
Gly Lys Ser Leu Glu Trp Ile Gly Asp Ile Asp Pro Asn Phe Asp Ser
45 50 55

TCC AGT TAC AAC CAG AAG TTC AAG GGA AAG GCC ACA TTG ACT GTA GAC 409
Ser Ser Tyr Asn Gln Lys Phe Lys Gly Lys Ala Thr Leu Thr Val Asp
60 65 70

AAG TCC TCC AAC ACA GCC TAC ATG GAG CTC CGC AGC CTG ACA TCT GAG 457
Lys Ser Ser Asn Thr Ala Tyr Met Glu Leu Arg Ser Leu Thr Ser Glu
75 80 85

GAC ACT GCA GTC TAT TAC TGT GCA AGA GGG GGA TTC CCC TAT GGT ATG 505
Asp Thr Ala Val Tyr Tyr Cys Ala Arg Gly Gly Phe Pro Tyr Gly Met
90 95 100 105

GAC TAC TGG GGT CAA GGA ACC TCA GTC ACC GTC TCC TCA GCC AAA ACG 553
Asp Tyr Trp Gly Gln Gly Thr Ser Val Thr Val Ser Ser Ala Lys Thr
110 115 120

ACA CCC CCA TCT GTC TAT CCA CTG GCC CCA AGCTT 588
Thr Pro Pro Ser Val Tyr Pro Leu Ala Pro
125 130

(2) INFORMATION ZU SEQ ID NO:3:

(i) SEQUENZCHARAKTERISTIKEN:
(A) LÄNGE: 41 Basenpaare
(B) TYP: Nukleinsäure
(C) STRANGFORM: Einzelstrang
(D) TOPOLOGIE: linear

(xi) SEQUENZDARSTELLUNG: SEQ ID NO:3:

CCCCGAATTC GAGGCTTCCC CACAAGCGAC CTACCACTGT T 41

(2) INFORMATION ZU SEQ ID NO:4:

(i) SEQUENZCHARAKTERISTIKEN:
(A) LÄNGE: 41 Basenpaare
(B) TYP: Nukleinsäure
(C) STRANGFORM: Einzelstrang
(D) TOPOLOGIE: linear

(xi) SEQUENZDARSTELLUNG: SEQ ID NO:4:

CCCCAAGCTT GGAAGATGGA TACAGTTGGT GCAGCATCAG C 41

(2) INFORMATION ZU SEQ ID NO:5:

(i) SEQUENZCHARAKTERSITIKEN:
(A) LÄNGE: 41 Basenpaare
(B) TYP: Nukleinsäure
(C) STRANGFORM: Einzelstrang
(D) TOPOLOGIE: linear

(xi) SEQUENZDARSTELLUNG: SEQ ID NO:5:

GGGGGAATTC CATACTGAGA AGAGCCTCTC CCACTCTCCT G 41

(2) INFORMATION ZU SEQ ID NO:6:

(i) SEQUENZCHARAKTERISTIKEN:
(A) LÄNGE: 41 Basenpaare
(B) TYP: Nukleinsäure
(C) STRANGFORM: Einzelstrang
(D) TOPOLOGIE: linear

(xi) SEQUENZDARSTELLUNG: SEQ ID NO:6:

GGGGAAGCTT GGGGCCAGTG GATAGACAGA TGGGGGTGTC G 41

(2) INFORMATION ZU SEQ ID NO:7:

(i) SEQUENZCHARAKTERISTIKEN:
(A) LÄNGE: 32 Basenpaare
(B) TYP: Nukleinsäure
(C) STRANGFORM: Einzelstrang
(D) TOPOLOGIE: linear

(xi) SEQUENZDARSTELLUNG: SEQ ID NO:7:

TGCCAAGCTT GCATGCCTGC AGGTCGAGAT CT 32

(2) INFORMATION ZU SEQ ID NO:8:

(i) SEQUENZCHARAKTERISTIKEN:
(A) LÄNGE: 57 Basenpaare
(B) TYP: Nukleinsäure
(C) STRANGFORM: Einzelstrang
(D) TOPOLOGIE: linear

(xi) SEQUENZDARSTELLUNG: SEQ ID NO:8:

CACGATATCA CCTCGAGCAC CTGGAAAATT AGGAAAAAAA AAAGTTTAGT AGGACAC 57

(2) INFORMATION ZU SEQ ID NO:9:

(i) SEQUENZCHARAKTERISTIKEN
(A) LÄNGE: 30 Basenpaare
(B) TYP: Nukleinsäure
(C) STRANGFORM: Einzelstrang
(D) TOPOLOGIE: linear

(xi) SEQUENZDARSTELLUNG: SEQ ID NO:9:

CCCGGTTACC GAGCTGATAC GTAAGTACAC 30

(2) INFORMATION ZU SEQ ID NO:10:

(i) SEQUENZCHARAKTERISTIKEN:
(A) LÄNGE: 21 Basenpaare
(B) TYP: Nukleinsäure
(C) STRANGFORM: Einzelstrang
(D) TOPOLOGIE: linear

(xi) SEQUENZDARSTELLUNG: SEQ ID NO:10:

TCTCTTGGAT GGTGACCATA G 21

(2) INFORMATION ZU SEQ ID NO:11:

(i) SEQUENZCHARAKTERISTIKEN:
(A) LÄNGE: 751 Basenpaare
(B) TYP: Nukleinsäure
(C) STRANGFORM: Einzelstrang
(D) TOPOLOGIE: linear

(xi) SEQUENZDARSTELLUNG: SEQ ID NO:11:

AGATCTTATA AAGTCAGTAT GATTTGCATA AATACATCTT CTTTTAGTGC TCCTGCCCCT 60

TAGAGTATTA TAAGACGATC TCTATCAGGA CAGTGTCATG AGCCACACAA ACTCAGGGAA 120

AGCTCGAAGA TGGTTTTCAC ACCTCAGATA CTTGGACTTA TGCTTTTTTG GATTTCAGGT 180

ATGACTGCTT GGACATGGAA ATAAAGTGGC GATAATATTT AAATATGAAA TATTCTTATT 240

TTATTTGAAA CCTAGCACCA CATTGTATTT GACTTCCTGT TTAAAGAAAT TAATACAATG 300

AAAGTCTTTT ATTTTTCTAA GTTGTTTAGG AGTKACTTAC ATATTCAATG TTGTGTCCTA 360

CTAAACTTTT TTTTTCCTAA TTTTCCAGGT GCTCGAGGTG ATATCGTGAT GACCCAGACT 420

CCACTCACTT TGTCGGGTTA CCGAGCTGAT ACGTAAGTAC ACTTTTCTCA TCTTTTTTTA 480

TGTGTAAGAC ACAGGTTTTC ATGTTAGGAG TTAAAGTCAG TTCAGAAAAT CTTGAGAAAA 540

TGGAGAGGGC TCATTATCAG TTGACGTGGC ATACAGTGTC AGATTTTCTG TTTATCAAGC 600

TAGTGAGATC AGGGGCAAAA AGAGGCTTTA GTTGAGAGGA AAGTAATTAA TACTATGGTC 660

ACCATCCAAG CGATTGGATC GGAGAATAAG CATGAATAGT TATTGAGATC TGGGTCTGAC 720

TGCAGGTAGC GTGGTCTTCT AGAGCGGCCG C 751

(2) INFORMATION ZU SEQ ID NO:12:

(i) SEQUENZCHARAKTERISTIKEN:
(A) LÄNGE: 23 Basenpaare
(B) TYP: Nukleinsäure
(C) STRANGFORM: Einzelstrang
(D) TOPOLOGIE: linear

(xi) SEQUENZDARSTELLUNG: SEQ ID NO:12:

AGCCTCTCGA GGTGACGTCT TGC 23

2) INFORMATION ZU SEQ ID NO:13:

(i) SEQUENZCHARAKTERISTIKEN:
(A) LÄNGE: 20 Basenpaare
(B) TYP: Nukleinsäure
(C) STRANGFORM: Einzelstrang
(D) TOPOLOGIE: linear

(xi) SEQUENZDARSTELLUNG: SEQ ID NO:13:

GTTTAATTTC CAGCTTGGTC 20

(2) INFORMATION ZU SEQ ID NO:14:

(i) SEQUENZCHARAKTERISTIKEN:
(A) LÄNGE: 30 Basenpaare
(B) TYP: Nukleinsäure
(C) STRANGFORM: Einzelstrang
(D) TOPOLOGIE: linear

(xi) SEQUENZDARSTELLUNG: SEQ ID NO:14:

TCACTGTCTC TGCACGTGAG TCCTAACTTC 30

(2) INFORMATION ZU SEQ ID NO:15:

(i) SEQUENZCHARAKTERISTIKEN:
(A) LÄNGE: 85 Basenpaare
(B) TYP: Nukleinsäure
(C) STRANGFORM: Einzelstrang
(D) TOPOLOGIE: linear

(xi) SEQUENZDARSTELLUNG: SEQ ID NO:15:

CCCCAAGCTT GAAGCAGTTC CATGAATACT CACCTTCCTG TGTTCTTTTC ACAGGTGTGC 60

TCAGCGAGGT CCAGCTGGAA TTCCC 85

(2) INFORMATION ZU SEQ ID NO:16:

(i) SEQUENZCHARAKTERISTIKEN:
(A) LÄNGE: 85 Basenpaare
(B) TYP: Nukleinsäure
(C) STRANGFORM: Einzelstrang
(D) TOPOLOGIE: linear

(xi) SEQUENZDARSTELLUNG: SEQ ID NO:16:

GGGAATTCCA GCTGGACCTC GCTGAGCACA CCTGTGAAAA GAACACAGGA AGGTGAGTAT 60

TCATGGAACT GCTTCAAGCT TGGGG 85

(2) INFORMATION ZU SEQ ID NO:17:

(i) SEQUENZCHARAKTERISTIKEN:
(A) LÄNGE: 735 Basenpaare
(B) TYP: Nukleinsäure
(C) STRANGFORM: Einzelstrang
(D) TOPOLOGIE: linear

(xi) SEQUENZDARSTELLUNG: SEQ ID NO:17:

GCGGCCGCAA TTCTCGAGGA TCCATTTCCT CAGAGAGTTA TTGGATTTGG ACTAGACTAT 60

CCTGCTGCTT GACCTATGTA CCTTTAAGTC CTTCCTCTCC AGCTTTTCTT CATTCGGATT 120

GGTTATTATA TACAAAGTCC CCTGGTCATG AATATGCAAA ATACCTAAGT CTATGGTAGC 180

TAAAAACAGG GATATCAACA CCCTGAAAAC AACATATCGA CGTTAGTCTT AAGGCACCAC 240

TGAGCCCAAG TCTTAGACAT CATGGCTTGG GTGTGGACCT TGCTTTTCCT GATGGCAGCT 300

GCCCAAAGTA AGACATCAGA AAAAAAGAGT TCCTAGGGAA ATTGAAGCAG TTCCATGAAT 360

ACTCACCTTC CTGTGTCCTT TGCACAGGTG TGCTCAGCGA GGTCCAGCTG GAATTCGAGC 420

TCCGCAGCCT GACATCTGAG GACTCTGCAG TCTATTACTG TGCAAGAGGG GGATTACACT 480

GGTTTCCTTA CTGGGGCCAA GGGACTCTGG TCACTGTCTC TGCACGTGAG TCCTAACTTC 540

TCCCATTCTA AATGCATGTT GGGGGGATTC TGGGCCTTCA GGACCAAGAT TCTCTGCAAA 600

CGGGAATCAA GATTCAACCC CTTTGTCCCA AAGTTGAGAC ATGGGTCTGG GTCAGGGACT 660

CTCTGCCTGC TGGTCTGTGG TGACATTAGA ACTGAAGTAT GATGAAGGAT CTGCCAGAAC 720

TGAAGCTGCG GCCGC 735

(2) INFORMATION ZU SEQ ID NO:18:

(i) SEQUENZCHARAKTERISTIKEN:
(A) LÄNGE: 26 Basenpaare
(B) TYP: Nukleinsäure
(C) STRANGFORM: Einzelstrang
(D) TOPOLOGIE: linear

(xi) SEQUENZDARSTELLUNG: SEQ ID NO:18:

GGGATATCAC GCGTACGGAT CCAACA 26

(2) INFORMATION ZU SEQ ID NO:19:

(i) SEQUENZCHARAKTERISTIKEN:
(A) LÄNGE: 28 Basenpaare
(B) TYP: Nukleinsäure
(C) STRANGFORM: Einzelstrang
(D) TOPOLOGIE: linear

(xi) SEQUENZDARSTELLUNG: SEQ ID NO:19:

TATGTTGGAT CCGTACGCGT CATATCCC 28

(2) INFORMATION ZU SEQ ID NO:20:

(i) SEQUENZCHARAKTERISTIKEN:
(A) LÄNGE: 28 Basenpaare
(B) TYP: Nukleinsäure
(C) STRANGFORM: Einzelstrang
(D) TOPOLOGIE: linear

(xi) SEQUENZDARSTELLUNG: SEQ ID NO:20:

TGCAGGTGTG CTCAGCGAGG TCCAGCTG 28

(2) INFORMATION ZU SEQ ID NO:21:

(i) SEQUENZCHARAKTERISTIKEN:
(A) LÄNGE: 20 Basenpaare
(B) TYP: Nukleinsäure
(C) STRANGFORM: Einzelstrang
(D) TOPOLOGIE: linear

(xi) SEQUENZDARSTELLUNG: SEQ ID NO:21:

CTGAGGAGAC GGTGACTGAG 20

## Patentansprüche

1. Verfahren zur Herstellung eines Immunglobulin-Expressionsvektors durch Klonierung eines DNA-Fragments, das einen für die komplette exprimierte V-Region eines ausgewählten Immunglobulins kodierenden Sequenzbereich enthält, in einen Basisvektor, der einen Promotor, die konstanten Immunglobulingen-Sequenzen und geeignete Restriktionsschnittstellen zur Insertion des oben genannten DNA-Fragments enthält, wobei die Klonierung derart erfolgt, daß ein Expressionsvektor mit einem operativen Immunglobulingen gebildet wird,
**dadurch gekennzeichnet,**
daß man zur Klonierung ein DNA-Fragment verwendet, das vor dem 5'-Ende des für die komplette exprimierte V-Region kodierenden Sequenzbereichs eine Region enthält, die für den vollständigen C-terminalen Abschnitt eines Immunglobulin-Leaderpeptids oder einen 3'-seitigen Teil davon kodiert und daß das Fragment direkt hinter dem 3'-Ende des für die komplette exprimierte V-Region kodierenden Sequenzbereichs endet.

2. Verfahren nach Anspruch 1,
**dadurch gekennzeichnet**,
daß man einen Basisvektor verwendet, der für konstante Regionen der schweren oder/und der leichten Kette kodierende Immunglobulingen-Sequenzen einschließlich der jeweiligen nicht-kodierenden Intronsequenzen enthält.

3. Verfahren nach einem der vorhergehenden Ansprüche,
**dadurch gekennzeichnet,**
daß das für die V-Region des Immunglobulingens kodierende DNA-Fragment durch folgende Schritte hergestellt wird:
(a) Isolierung von RNA aus einer Zelle, die das ausgewählte Immunglobulingen exprimiert, und Umschreiben der RNA in cDNA mit Hilfe einer Reversen Transkriptase,
(b) Zyklisierung der cDNA durch Ligation in verdünnter Lösung,
(c) Amplifizierung der zyklisierten cDNA oder eines Abschnitts davon mit Hilfe von 2 Primern (1) und (2), die aus der konstanten Region des ausgewählten Immunglobulingens stammen, wobei die Primer (1) und (2) so gewählt werden, daß das resultierende Amplifizierungsprodukt mindestens einen Abschnitt aus dem Exon 2 des Immunglobulingens, der für das C-terminale Ende eines Immunglobulin-Leaderpeptids kodiert, sowie die komplette exprimierte V-Region des ausgewählten Immunglobulingens enthält,
(d) vollständige oder teilweise Sequenzierung des Amplifizierungsprodukts aus (c),
(e) Konstruktion von 2 Primern (3) und (4) anhand der aus (d) gewonnenen Sequenzinformation, wobei der Primer (3) innerhalb der für den C-terminalen Abschnitt des Immunglobulin-Leaderpeptids kodierenden Region und der Primer (4) im Bereich des 3'-Endes der exprimierten V-Region an das Amplifizierungsprodukt aus (c) bindet,
(f) Amplifizierung des Amplifizierungsprodukts aus (c) oder einer entsprechenden RNA bzw. cDNA aus (a) mit Hilfe der Primer (3) und (4) und
(g) gegebenenfalls Restriktionsschnitt des Amplifizierungsprodukts aus (f) zur Erzeugung passender Enden für die Klonierung in den Basisvektor.

4. Verfahren nach Anspruch 3,
**dadurch gekennzeichnet**,
daß das Amplifizierungsprodukt aus Schritt (c) den 5'-untranslatierten Bereich des Immunglobulingens, den für das komplette Immunglobulin-Leaderpeptid kodierenden Bereich, den für die komplette exprimierte V-Region kodierenden Bereich sowie einen Teil des für die konstante Region des Immunglobulingens kodierenden Bereichs enthält.

5. Verfahren nach Anspruch 3 oder 4,
**dadurch gekennzeichnet**,
daß der zur Amplifizierung in Schritt (f) verwendete Primer (3) eine Erkennungssequenz für ein Restriktionsenzym enthält, durch deren Einbau keine funktionelle Veränderung der Aminosäuresequenz im Immunglobulin-Leaderpeptid erfolgt.

6. Verfahren nach Anspruch 5,
**dadurch gekennzeichnet**,
daß der Primer (3) bei Klonierung eines für die V-Region einer leichten Kette eines Immunglobulins kodierenden DNA-Fragments eine XhoI-Restriktionsschnittstelle enthält.

7. Verfahren nach Anspruch 5,
**dadurch gekennzeichnet**,
daß der Primer (3) bei Klonierung eines für die V-Region einer schweren Kette eines Immunglobulins kodierenden DNA-Fragments eine CelII-Restriktionsschnittstelle enthält.

8. Verfahren zur Gewinnung von Immunglobulinen,
**dadurch gekennzeichnet**,
daß man eine Zelle gleichzeitig oder schrittweise mit 2 kompatiblen Expressionsvektoren transformiert, die gemäß einem Verfahren nach einem der Ansprüche 1-7 hergestellt worden sind, positiv transformierte Zellen selektioniert, unter geeigneten Bedingungen kultiviert und Immunglobulin aus den Zellen oder dem Kulturmedium gewinnt, wobei einer der Expressionsvektoren ein für die schwere Kette eines ausgewählten Immunglobulins kodierendes Gen enthält und der andere Expressionsvektor ein für die leichte Kette dieses Immunglobulins kodierendes Gen enthält.

9. Verfahren zur Gewinnung von Immunglobulin,
**dadurch gekennzeichnet,**
daß man eine Zelle mit einem Expressionsvektor transformiert, der nach einem der Ansprüche 1-7 hergestellt worden ist, positiv transformierte Zellen selektioniert, unter geeigneten Bedingungen kultiviert und Immunglobulin aus den Zellen oder dem Kulturmedium gewinnt, wobei der Expressionsvektor sowohl ein für die schwere Kette eines ausgewählten Immunglobulins kodierendes Gen als auch ein für die leichte Kette dieses Immunglobulins kodierendes Gen enthält.

10. Verfahren nach Anspruch 8 oder 9,
**dadurch gekennzeichnet**,
daß man einen Expressionsvektor für die schwere Kette des Immunglobulins verwendet, der ein neo-Resistenzgen aufweist.

11. Verfahren nach Anspruch 10,
**dadurch gekennzeichnet**,
daß man einen Expressionsvektor für die leichte Kette des Immunglobulins verwendet, der kein neo-Resistenzgen aufweist.

12. Verfahren nach einem der Ansprüche 8 oder 10 bis 11,
**dadurch gekennzeichnet**,
daß man das Plasmid p16 026 als Expressionsvektor für die schwere Kette und das Plasmid p16 031 als Expressionsvektor für die leichte Kette verwendet.

13. Basisvektor zur Herstellung eines Expressionsvektors für eine Immunglobulin-Kette, der folgende essentielle Elemente enthält:
(a) einen zur Expression eines Immunglobulingens geeigneten Promotor,
(b) ein vom Exon 1 eines Immunglobulingen kodiertes Leaderpeptidsegment,
(c) das Intron 1 eines Immunglobulingens,
(d) eine Region, die für den vollständigen C-terminalen Abschnitt eines Immunglobulin-Leaderpeptids oder einen 5'-seitigen Teil davon kodiert,
(e) das komplette Intron 2 eines Immunglobulingens,
(f) eine komplette C-Region eines Immunglobulingens,
**dadurch gekennzeichnet**,
daß der Vektor weiterhin folgende Merkmale aufweist:
(g) eine Erkennungssequenz für ein Restriktionsenzym, die derartig lokalisiert ist, daß eine Spaltung des Vektors durch das Restriktionsenzym genau vor dem 5'-Ende von Intron 2 des Immunglobulingens stattfindet, und
(h) eine in die Region (d) eingeführte Erkennungssequenz für ein Restriktionsenzym, wobei die Erkennungssequenz mit der biologischen Funktion der Region (d) vereinbar ist.

14. Vektor nach Anspruch 13 zur Herstellung eines Expressionsvektors für sowohl die leichte als auch die schwere Immunglobulin-Kette,
**dadurch gekennzeichnet,**
daß er die essentiellen Elemente (a) bis (h) jeweils für die schwere Kette eines Immunglobulins und für die leichte Kette eines Immunglobulins aufweist.

15. Vektor nach Anspruch 13,
**dadurch gekennzeichnet**,
daß die Restriktionsstelle (g) eine BbrPI- oder SnaBI-Stelle ist.

16. Vektor nach Anspruch 13,
**dadurch gekennzeichnet**,
daß die Restriktionsstelle (h) eine CelII-Stelle ist.

17. Vektor nach Anspruch 13,
**dadurch gekennzeichnet,**
daß er p11315 oder ein Derivat davon ist.

18. Vektor nach Anspruch 13,
**dadurch gekennzeichnet**,
daß die Restriktionsstelle (h) eine XhoI-Stelle ist.

19. Vektor nach Anspruch 13
**dadurch gekennzeichnet,**
daß er p18023 oder ein Derivat davon ist.

20. Verfahren zur Herstellung eines therapeutischen oder/und diagnostischen Mittels,
**dadurch gekennzeichnet**,
daß man ein oder mehrere Immunglobuline, die gemäß einem Verfahren nach einem der Ansprüche 8 bis 12 gewonnen werden, sowie gegebenenfalls bekannte Hilfs-, Zusatz-, Träger- und Füllstoffe formuliert.
